# EUROPEAN PATENT SPECIFICATION

(11) **EP 4 087 499 B1**
(45) Date of publication and mention of the grant of the patent: **14.02.2024**
(21) Application number: 21819597.2
(22) Date of filing: 29.11.2021
(51) Int. Cl.: A61B 17/072, A61B 90/00

(54) **DEVICES FOR MANAGING ENERGY DISSIPATED WITHIN STERILE BARRIERS OF SURGICAL INSTRUMENT HOUSINGS**
VORRICHTUNGEN ZUR VERWALTUNG DER ENERGIE, DIE INNERHALB DER STERILEN BARRIEREN EINES CHIRURGISCHEN INSTRUMENTENGEHÄUSES ABGELEITET WIRD
DISPOSITIFS DE GESTION D'ÉNERGIE DISSIPÉE ENTRE DES BARRIÈRES STÉRILES DE BOÎTIERS D'INSTRUMENTS CHIRURGICAUX

(30) Priority: 02.12.2020 US 202017109589; 02.12.2020 US 202017109648
(43) Date of publication of application: 16.11.2022
(73) Proprietor: Cilag GmbH International, 6300 Zug (CH)
(72) Inventor: SHELTON, IV, Frederick E., Cincinnati, Ohio 45242 (US); FIEBIG, Kevin M., Cincinnati, Ohio 45242 (US); HARRIS, Demetrius N., Cincinnati, Ohio 45242 (US); MCELHANEY JR., Patrick G., Cincinnati, Ohio 45242 (US); LUO, Jesse, Cincinnati, Ohio 45242 (US); BOUDREAUX, Chad P., Cincinnati, Ohio 45242 (US)
(74) Representative: Carpmaels & Ransford LLP
(86) International application number: PCT/IB2021/061052
(87) International publication number: WO 2022/118162

(56) References cited:
- EP-A1- 3 187 125
- US-A1- 2016 106 424
- US-A1- 2016 310 134

## Description

### BACKGROUND

The present disclosure relates to surgical devices. More specifically, the present disclosure relates to handheld surgical systems for performing surgical procedures. US 2016/310134 discloses a surgical device configured for selective connection with an adapter, the adapter being configured for selective connection with end effectors or single use loading units. The surgical device comprises a power-pack and an outer shell housing, the outer shell housing having a shell cavity in which the power-pack is selectively situated, and the power-pack including an inner handle housing and an upper housing portion. The outer shell housing further comprises a plate assembly, the plate assembly including a plate rotatably supporting three coupling shafts. In use, when the adapter is mated to the surgical device, each of the coupling shafts couples with a corresponding rotatable connector sleeve of the adapter.

### SUMMARY

The invention is defined in claims 1 and 13. Any methods disclosed hereinafter are not explicitly recited by the claims (but are considered as useful for understanding the invention). The following summary is provided to facilitate an understanding of some of the innovative features unique to the aspects disclosed herein and is not intended to be a full description. A full appreciation of the various aspects can be gained by taking the entire specification, claims, and abstract as a whole.

In various aspects, a surgical instrument including a handheld device and an adapter assembly is disclosed. The handheld device includes an inner housing and a power source coupled to a first drive assembly including a first operating mode. The power source and first drive assembly are dispositioned within the inner housing. The adapter assembly includes an outer housing that defines an internal cavity. The outer housing is configured to encase the handheld device and further includes an energy management system configured to manage energy dissipated by the handheld device and a drive interface assembly including an internal interface and an external interface, wherein the internal interface is configured to mechanically couple to the first drive assembly of the handheld device. The surgical instrument further includes an interchangeable end effector including a second drive assembly. The second drive assembly includes a second operating mode that is different than the first operating mode of the first drive assembly, and the second drive assembly is configured to mechanically couple to the external interface of the drive interface. The internal interface of the drive interface assembly is configured to transfer a motion generated by the first drive assembly to the external interface of the drive interface assembly, and the external interface of the drive interface assembly is configured to transfer a motion of the inner interface of the drive interface assembly to the second drive assembly of the interchangeable end effector.

In various aspects, an adapter assembly configured to, at least partially, encase a handheld device of a surgical instrument configured for use with a plurality of interchangeable end effectors is disclosed. The handheld device includes a power source and a drive assembly. The adapter assembly can include an outer housing, including an internal cavity configured to encase the handheld device; a drive interface assembly, including an internal interface configured to mechanically engage the drive assembly of the handheld device; and an external interface configured to mechanically engage a drive assembly of an interchangeable end effector. The adapter assembly can further include an energy management system configured to manage energy dissipated by the handheld device when the surgical instrument is in use.

In various aspects, a surgical instrument including a handheld device and an adapter assembly is disclosed. The handheld device includes a first drive assembly and a power source. The adapter assembly includes an internal cavity configured to accommodate a handheld device, wherein the adapter assembly is configured to establish a sterile barrier around the handheld device, and an energy management system configured to extract energy dissipated by the handheld device from the internal cavity without disrupting the sterile barrier.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### FIGURES

The features of various aspects are set forth with particularity in the appended claims. The various aspects, however, both as to organization and methods of operation, together with further objects and advantages thereof, may best be understood by reference to the following description, taken in conjunction with the accompanying drawings as follows.
FIG. 1 illustrates a perspective view of a surgical instrument that includes an adapter assembly configured to create a sterile barrier around a handheld surgical device and energy management components, in accordance with at least one non-limiting aspect of the present disclosure.
FIG. 2 illustrates a sectioned perspective view of a handheld assembly configured to be encased within the adapter assembly of the surgical instrument of FIG. 1.
FIG. 3 illustrates a perspective view of the adapter assembly and handheld device of the surgical instrument of FIG. 1.
FIG. 4 illustrates a perspective view of the adapter assembly and handheld device of the surgical instrument of FIG. 1.
FIG. 5 illustrates a perspective assembly view of an adapter assembly that includes energy management components, in accordance with at least one non-limiting aspect of the present disclosure.
FIG. 6 illustrates a perspective back view of the adapter assembly of FIG. 5.
FIGS. 7A and 7B illustrate sectioned front views of a handheld surgical device being installed into the adapter assembly of FIGS. 5 and 6.
FIG. 8 illustrates a sectioned side view of an adapter assembly that includes energy management components, in accordance with at least one non-limiting aspect of the present disclosure.
FIG. 9 illustrates a sectioned side view of a surgical instrument that includes energy management components, in accordance with at least one non-limiting aspect of the present disclosure.
FIGS. 10A and 10B illustrate sectioned top views of the surgical instrument of FIG. 9.
FIGS. 11A and 11B illustrate top views of an energy management component of the adapter assembly of FIG. 9.
FIG. 12 illustrates a chart depicting a variable rate of energy management implemented by the surgical instrument of FIG. 9.
FIG. 13 illustrates a sectioned side view of a surgical instrument including a handheld surgical device and an adapter assembly that includes energy management components, in accordance with at least one non-limiting aspect of the present disclosure.
FIG. 14 illustrates a side view of an energy management component of the surgical instrument of FIG. 13.
FIG. 15 illustrates a sectioned side view of a surgical instrument including a handheld surgical device and an adapter assembly with energy management components, in accordance with at least one non-limiting aspect of the present disclosure.
FIG. 16 illustrates a sectioned side view of a surgical instrument including a handheld surgical device and an adapter assembly that includes and energy management system, in accordance with at least one non-limiting aspect of the present disclosure.
FIG. 17 illustrates a sectioned side view of the energy management system of the handheld device and adapter assembly of FIG. 16.
FIG. 18 illustrates a sectioned side view of an energy management component of the energy management system of FIG. 17.
FIG. 19 illustrates a side view of another energy management component of the energy management system of FIG. 16.
FIG. 20 illustrates a sectioned perspective view of a surgical instrument including a handheld surgical device and a distal portion of an adapter assembly with energy management components, in accordance with at least one non-limiting aspect of the present disclosure.
FIG. 21 illustrates a sectioned perspective view of an energy management component of the adapter assembly of FIG. 20.
FIG. 22 illustrates a perspective view of another energy management component of the adapter assembly of FIG. 20.
FIG. 23 illustrates a sectioned side view of a surgical instrument including a handheld surgical device and an adapter assembly that includes an energy management system, in accordance with at least one non-limiting aspect of the present disclosure.
FIG. 24 illustrates a sectioned perspective view of the energy management component of the surgical instrument of FIG. 23.
FIG. 25 illustrates a sectioned perspective view of another energy management component of an energy management system of a surgical instrument, in accordance with at least one non-limiting aspect of the present disclosure.
FIG. 26 illustrates a sectioned perspective view of a surgical instrument including an energy management system, in accordance with at least one non-limiting aspect of the present disclosure.
FIG. 27 illustrates a chart depicting an energy response of the energy management system of FIG. 26.
FIG. 28 illustrates a sectioned perspective view of an adapter assembly of a surgical instrument that includes an energy management component, in accordance with at least one non-limiting aspect of the present disclosure.
FIGS. 29A and 29B illustrate sectioned profile views of energy management components of the adapter assembly of FIG. 28.
FIGS. 30A-30C collectively illustrate various views of energy management systems and a chart depicting an energy response of the illustrated energy management systems, in accordance with at least one non-limiting aspect of the present disclosure.
FIG. 31 illustrates a perspective view of an energy management system of a surgical instrument, in accordance with at least one non-limiting aspect of the present disclosure.
FIG. 32 illustrates a sectioned perspective view of an energy management system of a surgical instrument, in accordance with at least one non-limiting aspect of the present disclosure.
FIG. 33 illustrates a sectioned front view of the energy management system of FIG. 32.
FIG. 34 illustrates a schematic of a control circuit configured to manage energy dissipated by a surgical instrument, in accordance with at least one non-limiting aspect of the present disclosure.

Corresponding reference characters indicate corresponding parts throughout the several views. The exemplifications set out herein illustrate certain embodiments of the invention, in one form, and such exemplifications are not to be construed as limiting the scope of the invention in any manner.

### DESCRIPTION

Applicant of the present application also owns the following U.S. Patent Applications that were filed on even date herewith:
- U.S. Patent Application entitled METHOD FOR TISSUE TREATMENT BY SURGICAL INSTRUMENT; Attorney Docket No. END9291USNP1/200802-1M;
- U.S. Patent Application entitled SURGICAL INSTRUMENTS WITH INTERACTIVE FEATURES TO REMEDY INCIDENTAL SLED MOVEMENTS; Attorney Docket No. END9291USNP2/200802-2;
- U.S. Patent Application entitled SURGICAL INSTRUMENTS WITH SLED LOCATION DETECTION AND ADJUSTMENT FEATURES; Attorney Docket No. END9291USNP3/200802-3;
- U.S. Patent Application entitled SURGICAL INSTRUMENT WITH CARTRIDGE RELEASE MECHANISMS; Attorney Docket No. END9291USNP4/200802-4;
- U.S. Patent Application entitled DUAL-SIDED REINFORCED RELOAD FOR SURGICAL INSTRUMENTS; Attorney Docket No. END9291USNP5/200802-5;
- U.S. Patent Application entitled SURGICAL SYSTEMS WITH DETACHABLE SHAFT RELOAD DETECTION; Attorney Docket No. END9291USNP6/200802-6;
- U.S. Patent Application entitled SURGICAL INSTRUMENTS WITH ELECTRICAL CONNECTORS FOR POWER TRANSMISSION ACROSS STERILE BARRIER; Attorney Docket No. END9291USNP7/200802-7;
- U.S. Patent Application entitled POWERED SURGICAL INSTRUMENTS WITH EXTERNAL CONNECTORS; Attorney Docket No. END9291USNP9/200802-9;
- U.S. Patent Application entitled POWERED SURGICAL INSTRUMENTS WITH SMART RELOAD WITH SEPARATELY ATTACHABLE EXTERIORLY MOUNTED WIRING CONNECTIONS; Attorney Docket No. END9291USNP10/200802-10;
- U.S. Patent Application entitled POWERED SURGICAL INSTRUMENTS WITH COMMUNICATION INTERFACES THROUGH STERILE BARRIER; Attorney Docket No. END9291USNP1 1/200802-11; and
- U.S. Patent Application entitled POWERED SURGICAL INSTRUMENTS WITH MULTI-PHASE TISSUE TREATMENT; Attorney Docket No. END9291USNP12/200802-12.

Applicant of the present application owns the following U.S. Patent Applications, filed on December 4, 2018:
- U.S. Patent Application Serial No. 16/209,385, entitled METHOD OF HUB COMMUNICATION, PROCESSING, STORAGE AND DISPLAY;
- U.S. Patent Application Serial No. 16/209,395, entitled METHOD OF HUB COMMUNICATION;
- U.S. Patent Application Serial No. 16/209,403, entitled METHOD OF CLOUD BASED DATA ANALYTICS FOR USE WITH THE HUB;
- U.S. Patent Application Serial No. 16/209,407, entitled METHOD OF ROBOTIC HUB COMMUNICATION, DETECTION, AND CONTROL;
- U.S. Patent Application Serial No. 16/209,416, entitled METHOD OF HUB COMMUNICATION, PROCESSING, DISPLAY, AND CLOUD ANALYTICS;
- U.S. Patent Application Serial No. 16/209,423, entitled METHOD OF COMPRESSING TISSUE WITHIN A STAPLING DEVICE AND SIMULTANEOUSLY DISPLAYING THE LOCATION OF THE TISSUE WITHIN THE JAWS;
- U.S. Patent Application Serial No. 16/209,427, entitled METHOD OF USING REINFORCED FLEXIBLE CIRCUITS WITH MULTIPLE SENSORS TO OPTIMIZE PERFORMANCE OF RADIO FREQUENCY DEVICES;
- U.S. Patent Application Serial No. 16/209,433, entitled METHOD OF SENSING PARTICULATE FROM SMOKE EVACUATED FROM A PATIENT, ADJUSTING THE PUMP SPEED BASED ON THE SENSED INFORMATION, AND COMMUNICATING THE FUNCTIONAL PARAMETERS OF THE SYSTEM TO THE HUB;
- U.S. Patent Application Serial No. 16/209,447, entitled METHOD FOR SMOKE EVACUATION FOR SURGICAL HUB;
- U.S. Patent Application Serial No. 16/209,453, entitled METHOD FOR CONTROLLING SMART ENERGY DEVICES;
- U.S. Patent Application Serial No. 16/209,458, entitled METHOD FOR SMART ENERGY DEVICE INFRASTRUCTURE;
- U.S. Patent Application Serial No. 16/209,465, entitled METHOD FOR ADAPTIVE CONTROL SCHEMES FOR SURGICAL NETWORK CONTROL AND INTERACTION;
- U.S. Patent Application Serial No. 16/209,478, entitled METHOD FOR SITUATIONAL AWARENESS FOR SURGICAL NETWORK OR SURGICAL NETWORK CONNECTED DEVICE CAPABLE OF ADJUSTING FUNCTION BASED ON A SENSED SITUATION OR USAGE;
- U.S. Patent Application Serial No. 16/209,490, entitled METHOD FOR FACILITY DATA COLLECTION AND INTERPRETATION; and
- U.S. Patent Application Serial No. 16/209,491, entitled METHOD FOR CIRCULAR STAPLER CONTROL ALGORITHM ADJUSTMENT BASED ON SITUATIONAL AWARENESS.

Before explaining various aspects of surgical devices and generators in detail, it should be noted that the illustrative examples are not limited in application or use to the details of construction and arrangement of parts illustrated in the accompanying drawings and description. The illustrative examples may be implemented or incorporated in other aspects, variations, and modifications, and they may be practiced or carried out in various ways. Further, unless otherwise indicated, the terms and expressions employed herein have been chosen for the purpose of describing the illustrative examples for the convenience of the reader and are not for the purpose of limitation thereof. Also, it will be appreciated that one or more of the following described aspects, expressions of aspects, and/or examples, can be combined with any one or more of the other following described aspects, expressions of aspects, and/or examples.

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

According to some non-limiting aspects of the present disclosure, surgical instruments can include handle assemblies that are configured to accommodate a variety of interchangeable tools, such as end effectors and/or single-use loading units (SLUs), among others. As such, the surgical instruments disclosed herein can provide increased versatility and, thus, value for implementing clinicians. However, not all surgical instruments and end effectors are configured to operate in the same way. For example, according to one non-limiting aspect of the present disclosure, a surgical instrument can employ a rotational transmission of power and an interchangeable tool (e.g., an end effector) can be configured for linear actuation. The surgical instrument configured to employ a rotational transmission of power would thus be incompatible with the linear driven end effector and, thus, its versatility and value would be diminished.

Certain surgical instruments are known to address the aforementioned incompatibilities, such as the surgical instrument described in U.S. Patent No. 10,603,128, entitled HANDHELD ELECTROMECHANICAL SURGICAL SYSTEM, granted March 31, 2020. Such surgical instruments utilize a specifically configured outer shell housing, which includes one or more interfacing components configured to selectively transfer rotational forces from motors of the surgical instrument to an adaptor of a connected end effector. Although the outer shell houses the aforementioned components, it must inherently encompass the surgical instrument to effectively interface the surgical instrument with any interchangeable tool, thereby facilitating the enhanced versatility of the surgical instrument. The outer shell housing is of increased importance due to the sterilization requirements of operating rooms that the surgical instruments are typically used in.

It is axiomatic that strict sterilization of the operating room and surgical equipment is required during any surgery. The strict hygiene and sterilization conditions required in an operating room necessitate the highest possible sterility of all medical devices and equipment. Part of that sterilization process is the need to sterilize anything that comes in contact with the patient or penetrates the sterile field, such as the surgical instrument, including its end effector, adapter assembly, and requisite components. Aside from the aforementioned adapter assemblies being configured to transfer rotational forces from motors of the surgical instrument to an adaptor of a connected end effector, the outer shell of such adapter assemblies can be configured to prevent contaminants from adversely effecting the sterile barrier.

However, the handheld devices encased in the outer housing often include a power pack, a motor assembly, and/or a control assembly among other electromechanical subassemblies. Each of these subassemblies can generate energy (e.g., thermal, vibrational, acoustic) that can adversely effect the environment the surgical instrument is expected to function in. These environments are contained when the handheld surgical device is encased within the outer housing, especially since the outer housing is typically configured to create a sterile barrier between the operating room and the handheld surgical device. Thus, although encasing a handheld surgical device can enhance versatility and sterility, it can also result in instrument failure, decreased life, and/or hazardous operating conditions. Accordingly, the surgical instruments disclosed herein are specifically configured to accommodate adaptors of a wide variety of interchangeable tools while responsibly managing the environmental conditions in which the surgical instrument is expected to function. As such, the disclosed surgical instruments are versatile, longer lasting, and more reliable than existing surgical instruments.

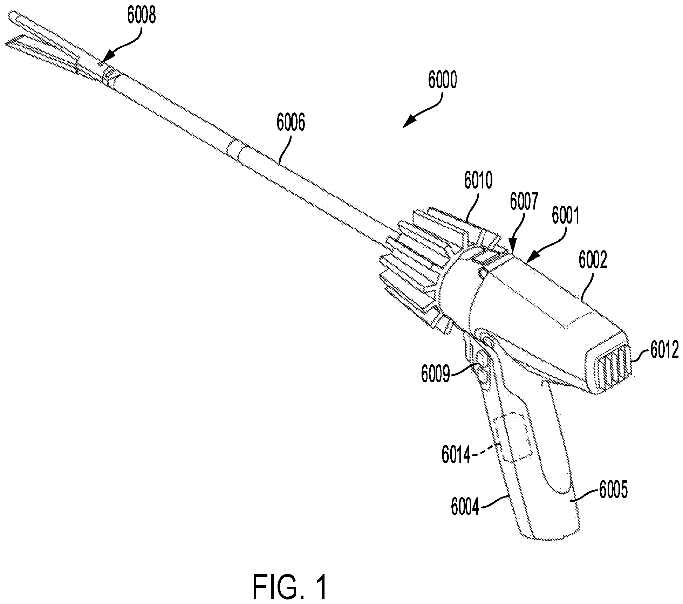

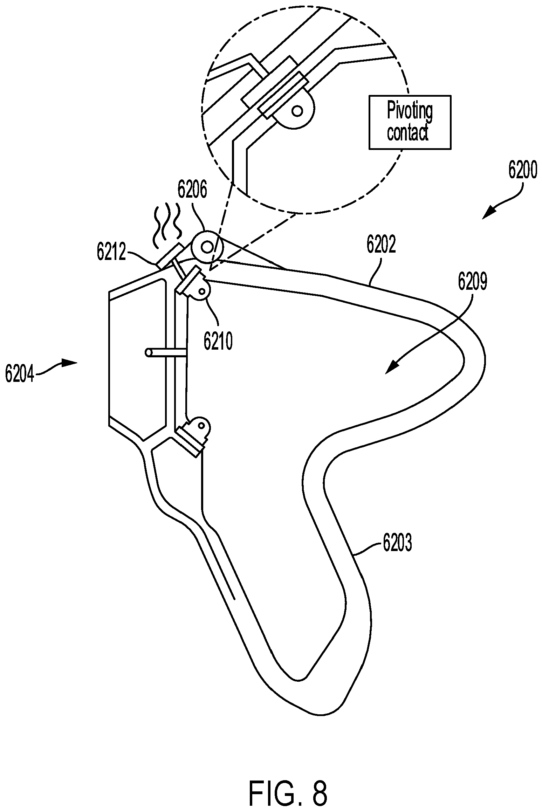

Referring now to FIG. 1, a perspective view of a surgical instrument 6000 that includes an adapter assembly 6001 configured to create a sterile barrier around a handheld surgical device with energy management components 6010, 6012, 6014 is depicted in accordance with at least one non-limiting aspect of the present disclosure. According to the non-limiting aspect of FIG. 1, the adapter assembly 6001 can include a proximal portion 6002 and a distal portion 6004 connected in a clamshell configuration via a hinge 6007. Collectively, the proximal portion 6002 and the distal portion 6004 can constitute an outer shell or housing that defines an internal cavity configured to encase a handheld device that can generate energy when the surgical instrument is in use. Accordingly, the adapter assembly 6001 is configured to transition from an open configuration, wherein the hinge 6007 is open and the sterile barrier is disrupted, to a closed configuration, as seen in FIG. 8, wherein the hinge 6206 is closed and the sterile barrier is established. The proximal portion 6202 can include a handle portion 6203 configured for the ergonomic handling of the surgical instrument when the handheld device is installed within the adapter assembly 6200. As also can be seen in FIG. 8, the proximal portion 6202 and distal portion 6204 of the adapter assembly 6200 can each include energy management components 6210, 6212 configured to effectively manage energy created by a handheld device when installed within an internal cavity 6209 of the adapter assembly 6200.

Still referring to FIG. 1, the adapter assembly 6001 of the surgical instrument 6000 can be configured to accommodate a variety of interchangeable shaft assemblies 6006 and end effectors 6008. In other words, the surgical instrument 6000 is configured for selective attachment thereto of a plurality of different end effectors 6008 that are each configured for actuation and manipulation by the powered handheld electromechanical surgical instrument 6000. As such, the adapter assembly 6001 can include a drive assembly configured to engage with a drive assembly of a handheld device encased within the internal cavity of the adapter assembly 6001. Likewise, the adapter assembly 6001 can include external buttons 6009 configured to engage with buttons of the handheld device encased within, while preserving the sterile barrier. Additionally, the drive assembly can be mechanically configured to translate forces generated by the drive assembly of the handheld assembly to the drive assembly of the interchangeable shaft and/or end effector. The drive assembly of the handheld device can include one or more motors that can generate energy (e.g., thermal, vibrational, and acoustic) when the surgical instrument 6000 is in use. However, because the adapter assembly 6001 is also configured to establish a sterile barrier around the handheld device, the dissipated energy can be trapped. Accordingly, the energy management components 6010, 6012, 6014 can assist in the effective management and dissipation of energy dissipated by the handheld device.

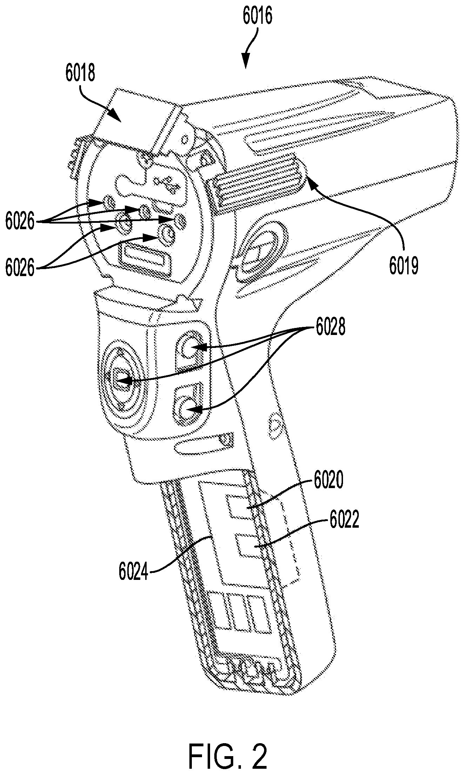

Referring now to FIG. 2, a sectioned perspective view of a handheld device 6016 configured to be encased within the adapter assembly 6001 of the surgical instrument 6000 of FIG. 1 is depicted in accordance with at least one aspect of the present disclosure. According to the non-limiting aspect of FIG. 2, the handheld device can further include energy management components 6018, 6019, 6020, 6022, 6024. Additionally, FIG. 2 a plurality of interface components 6026 of the drive assembly of the handheld device 6016 can be dispositioned on a forward-facing surface of the handheld device 6016. The interface components 6026 can mechanically engage corresponding interface components of the adapter assembly 6001 (FIG. 1) such that activation of the drive assembly of the handheld device 6016 can translate forces to the interchangeable shaft assembly 6006 (FIG. 1) and end effector 6008 (FIG. 1). It shall be appreciated that, through the use of the adapter assembly 6001 and the plurality of interface components 6026, the handheld device 6016 can be reused with versatility. Additionally, the handheld device 6016 can include a plurality of function buttons 6028, which can be configured to engage the external buttons 6009 (FIG. 1) of the adapter assembly 6001 (FIG. 1), such that a user can activate them without disrupting the sterile barrier.

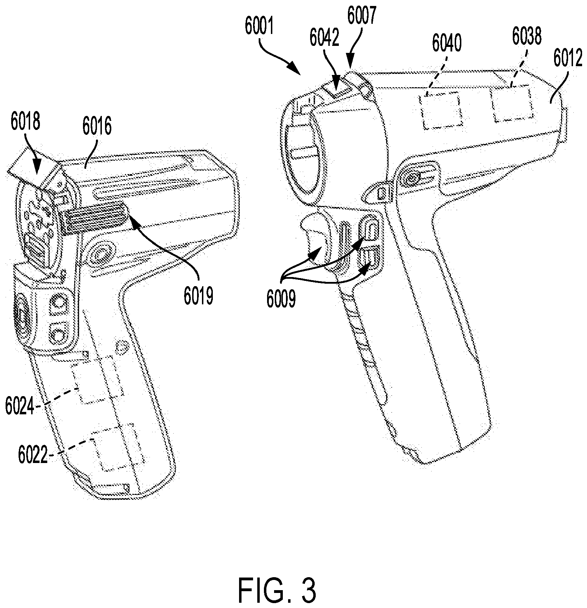

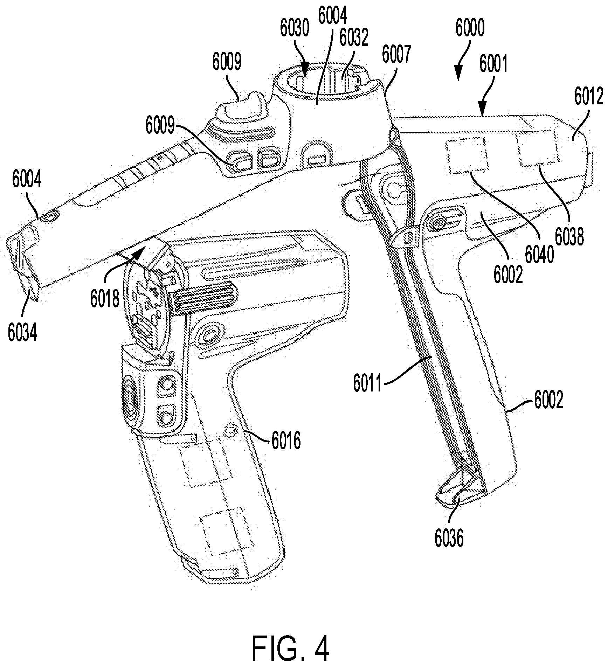

Referring now to FIGS. 3 and 4, perspective views of the adapter assembly 6001 and handheld device 6016 of the surgical instrument 6000 of FIG. 1 are depicted in accordance with at least one aspect of the present disclosure. According to the non-limiting aspect of FIG. 3, the relative size of the handheld device 6016 can be specifically configured such that it can be encased within an internal cavity of the adapter assembly 6001. It shall be appreciated that geometrical energy management components 6012, 6038, 6040, 6042 of the adapter assembly 6001 can mechanically engage corresponding energy management components 6018, 6019, 6022, 6024 of the handheld device 6016 when the handheld device 6016 is properly installed within the internal cavity of the adapter assembly 6001. Accordingly, energy dissipated by the handheld device 6016 can be effectively managed by the mechanical engagement of the energy management components 6012, 6038, 6040, 6042 of the adapter assembly 6001 and the corresponding energy management components 6018, 6019, 6022, 6024 of the handheld device 6016. In the non-limiting aspect of FIG. 3, the hinge 6007 of the adapter assembly 6001 can be positioned in a closed configuration, thereby establishing a sterile barrier between the ambient environment of the operating room in which it is used and an internal cavity configured to encase the handheld device 6016.

According to the non-limiting aspect of FIG. 4, the hinge 6007 of the adapter assembly 6001 can be positioned in an open configuration, exposing the internal cavity 6011 such that the handheld device 6016 can be properly installed. Additional features such as corresponding male 6034 and female 6036 components of a clasping lock can be included to enhance the sterile barrier, thereby fortifying the adapter assembly 6001 from being inadvertently opened and exposed to the non-sterile environment. Once again, it is evident how the energy management components 6012, 6038, 6040, 6042 (FIG. B3) of the adapter assembly 6001 can be configured to engage the corresponding energy management components 6018, 6019 (FIG. 2), 6022 (FIG. 2), 6024 (FIG. 2) of the handheld device 6016 upon proper installation. The energy management systems and components will be further discussed in detail. However, it shall be appreciated that the non-limiting aspect of FIGS. 1-4 are only presented for illustrative purposes. Accordingly, other non-limiting aspects contemplated by the present disclosure include any number of the following energy management components and systems in any combination, to accomplish a desired means of energy management when the surgical instrument is in use.

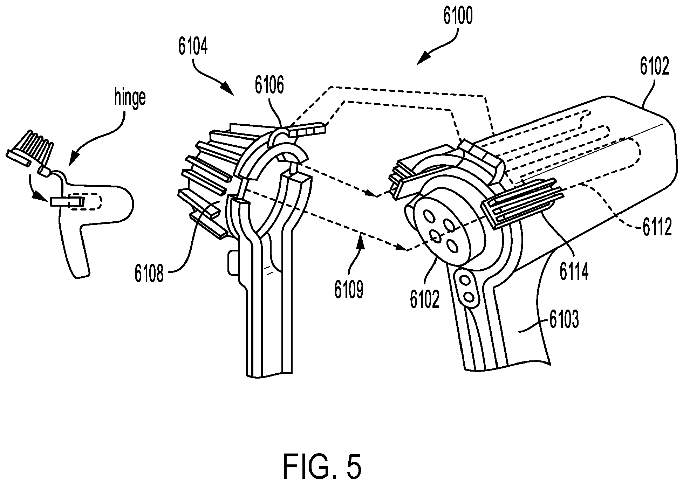

Referring now to FIG. 5, a perspective front view of the adapter assembly 6100 of FIG. 5 is depicted in accordance with at least one non-limiting aspect of the present disclosure. According to the non-limiting aspect of FIG. 5, the adapter assembly 6100 can include a proximal portion 6102 and a distal portion 6104 connected in a clamshell configuration via a hinge 6106. Collectively, the proximal portion 6102 and the distal portion 6104 can constitute an outer shell or housing configured to encase a handheld device including one or more motors 6112. The proximal portion 6102 can include a handle portion 6103 configured for ergonomic handling of the surgical instrument when the handheld device is installed within the adapter assembly 6100. As can be seen in FIG. 5, the proximal portion 6102 and distal portion 6104 of the adapter assembly 6100 can each include energy management components 6108, 6114 configured to effectively manage energy created by a handheld device when installed within an internal cavity 6109 of the adapter assembly 6100.

In further reference to FIG. 5, the proximal portion 6102 of the adapter assembly 6100 can be dimensionally configured to accommodate one or more motors 6112 of the handheld device. As previously discussed, the adapter assembly 6100 can be configured as a sterile barrier that can protect the handheld device from the non-sterile environment of the operating room. Thus, the adapter assembly 6100 of FIG. 5 can be structurally sealed, thereby capable of preventing contaminants from the operating environment from accessing an internal cavity 6109 of the adaptor assembly 6100 and, thus, preventing the reuse of the handheld device stored within. However, the one or more motors 6112 of the handheld device can produce energy (e.g., thermal, vibration, acoustical) when the surgical instrument is in use. Because the adapter assembly 6100 of FIG. 5 can be structurally sealed, it not only prevents contaminants from accessing the internal cavity 6109, but it also prevents energy (e.g., thermal, vibration, acoustical) that is generated during use from escaping the internal cavity 6109. Accordingly, the adapter assembly 6100 can include several energy management components 6108, 6114 to assist the release of energy (e.g., thermal, vibration, acoustical) from the internal cavity 6109.

Still referring to FIG. 5, the adapter assembly 6100 can include a first heat sink 6108 on the distal portion 6104. The first heat sink 6108 can be configured to remove thermal energy dissipated by the one or more motors 6112 from the internal cavity 6109 of the adapter assembly 6100. The first heat sink 6108 can be configured to mechanically contact thermally conductive channels 6114, which include a surface area within the internal cavity 6109. For example, the first heat sink 6108 can be configured to mechanically engage a thermally conductive channel 6114 when the distal portion 6104 engages the proximal portion 6102 of the adapter assembly 6100, thereby creating a sterile barrier. However, because the first heat sink 6108 remains in thermal communication with the internal cavity 6109 via the thermally conductive channel 6114, the first heat sink 6108 can still remove dissipated thermal energy dissipated in the internal cavity 6109 of the adapter assembly 6100. Thus, even though contaminants cannot enter the internal cavity 6109 of the adapter assembly 6100, thermal energy can escape the internal cavity 6109 of the adapter assembly 6100. In the non-limiting aspect of FIG. 5, the thermally conductive channel 6114 can include an external heat sink, which supplements the heat transfer capabilities of the first heat sink 6108.

In some non-limiting aspects, the adapter assembly 6100 of FIG. 5 can include thermally conductive channels 6114 that can be placed in mechanical contact with the 6112 motors themselves, thereby improving the thermally conductive path from the energy source and enhancing the efficiency of the thermally conductive channel 6114. According to such aspects, the thermally conductive channel 6114 can eliminate the radiative means of heat transfer and can provide a more efficient, conductive path to the first heat sink 6108. Alternatively and/or additionally, the thermally conductive channel 6114 can be placed in mechanical contact with a specifically configured surface area within the internal cavity 6109. For example, a portion of an inner wall of the internal cavity 6109 can be configured as part of the thermally conductive channel 6114. Since the efficiency of the thermally conductive channel 6114 can improve as the surface area increases, this can enhance the removal of thermal energy from the internal cavity 6109. Accordingly, the radiative means of heat transfer can be inherently more efficient due to the increased surface area. Although the non-limiting aspect of FIG. 5 includes a first and second heat sink 6108, 6110 (FIG. 1), it shall be appreciated that the present disclosure contemplates other non-limiting aspects wherein any number of heat sinks, channels, and baffles are used to establish similar paths by which generated thermal energy can escape the internal cavity 6109.

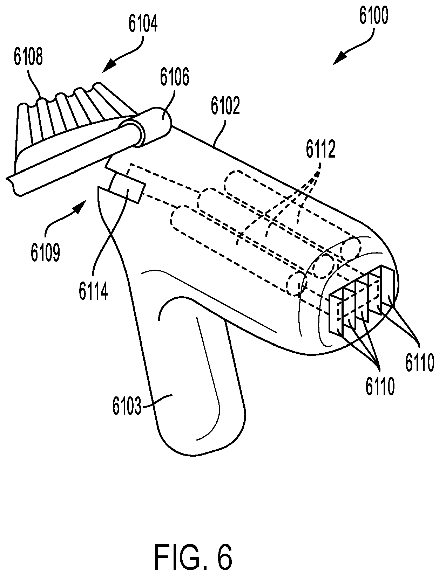

Referring now to FIG. 6, a perspective view of the back of the adapter assembly 6100 of FIG. 5 is depicted in accordance with at least one non-limiting aspect of the present disclosure. According to the non-limiting aspect of FIG. 6, the adapter assembly 6100 can further include a second heat sink 6110 coupled to the proximal portion 6102, in close proximity to the one or more motors 6112. The second heat sink 6110 can also be coupled to a thermally conductive channel, thereby enabling it to remove thermal energy produced by the one or more motors 6112 from the internal cavity 6109 without disturbing the sterile barrier created by adapter assembly 6100. In other non-limiting aspects, the second heat sink 6110 can be directly coupled to the one or more motors 6112, which can provide a more efficient, conductive path to the second heat sink 6110. Although the non-limiting aspect of FIGS. 5 and B6 depict a first heat sink 6108 and a second sink 6110 that are passive and include a plurality of integrally formed fins, the present disclosure contemplates other non-limiting aspects wherein any number of heat sink configurations can be implemented to enhance energy management within the adapter assembly 6100. For example, the adapter assembly 6100 can include active heat sinks, stamped heat sinks, bonded-formed heat sinks, and/or the like.

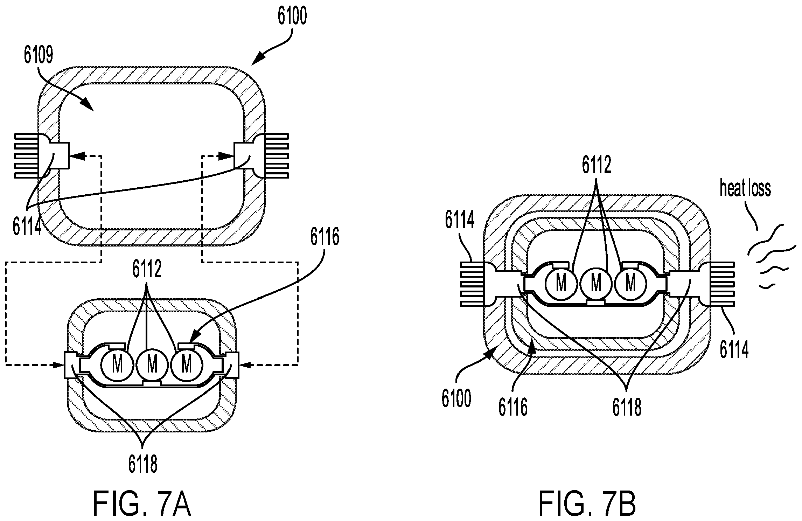

Referring now to FIGS. 7A and 7B, a sectioned front view of a handheld surgical device installed in the adapter assembly of FIGS. 5 and 6 is depicted in accordance with at least one non-limiting aspect of the present disclosure. According to the non-limiting aspect of FIG. 7A, the adapter assembly 6100 of FIGS. 5 and 6 is shown in more detail. Specifically, the thermally conductive channels 6114 are clearly depicted as defining a thermal path from the internal cavity 6109 to the exterior of the adapter assembly 6100. Accordingly, the thermally conductive channels 6114 enable the adapter assembly 6100 to preserve the sterile barrier, thereby protecting the contents of its internal cavity 6109 from external contamination. FIG. 7A also depicts the handheld device 6116, including three motors 6112, although other non-limiting aspects include handheld devices 6116 with any number of motors. Two conductive contacts 6118 are also depicted as configured to mechanically contact each of the three motors 6112. The conductive contacts 6118 are likewise configured to mechanically contact the thermally conductive channels 6114 when the handheld device 6116 is properly installed within the internal cavity 6109 of the adapter assembly 6100.

According to the non-limiting aspect of FIG. 7B, the handheld device 6116 has been properly installed within the internal cavity 6109 of the adapter assembly 6100. The conductive contacts 6118 of the handheld device 6116 can be in mechanical contact with the thermally conductive channels 6114, establishing a direct conductive path from the motors 6112 to an exterior of the adapter assembly 6100. When the surgical instrument is in use and the motors 6112 are generating thermal energy, the resulting thermal energy can travel through the conductive contacts 6118 into the thermally conductive channels 6114 and into the fins of the external heat sinks, where it can be safely convected away from the surgical instrument and into the operating room. Accordingly, generated thermal energy will not remain within the internal cavity 6109 of the adapter assembly 6100, and the surgical instrument will be at less of a risk of overheating, and thus, the damage and/or dangers associated with overheating.

Referring now to FIG. 8, a sectioned side view of an adapter assembly 6200 that includes energy management components 6210, 6212 is depicted in accordance with at least one non-limiting aspect of the present disclosure. According to the non-limiting aspect of FIG. 8, the adapter assembly 6200 can include a proximal portion 6202 and a distal portion 6204 connected in a clamshell configuration via a hinge 6206. Collectively, the proximal portion 6202 and the distal portion 6204 can constitute an outer shell or housing that defines an internal cavity 6209 configured to encase a handheld device that can generate energy when the surgical instrument is in use. Accordingly, the adapter assembly 6200 is configured to transition from an open configuration, wherein the hinge 6206 is open and the sterile barrier is disrupted, to a closed configuration, wherein the hinge 6206 is closed and the sterile barrier is established. The proximal portion 6202 can include a handle portion 6203 configured for the ergonomic handling of the surgical instrument when the handheld device is installed within the adapter assembly 6200. As can be seen in FIG. 8, the proximal portion 6202 and distal portion 6204 of the adapter assembly 6200 can each include energy management components 6210, 6212 configured to effectively manage energy created by a handheld device when installed within an internal cavity 6209 of the adapter assembly 6200.

Still referring to FIG. 8, the adapter assembly 6200 can include a pivoting contact 6210 on the proximal portion 6202. The pivoting contact 6210 can be configured to mechanically contact a thermally conductive surface area 6212 dispositioned on the distal portion 6204 of the adapter assembly 6200 when the clamshell outer housing is closed. Accordingly, the pivoting contact 6210 and thermally conductive surface area 6212 can establish a thermally conductive path when the clamshell outer housing is closed, wherein the thermally conductive path is configured to remove thermal energy generated from the internal cavity 6209 of the adapter assembly 6200. The pivoting contact 6210 can be pivotally coupled to the proximal portion 6202 of the adapter assembly 6200 and therefore, configured to optimize mechanical contact with the thermally conductive surface area 6212. For example, the thermally conductive path can be improved based on the degree of contact established between the pivoting contact 6210 and the thermally conductive surface area 6212. As previously discussed, the hinge 6206 facilitates motion between the proximal portion 6202 and distal portion 6204 as the adapter assembly 6200 transitions from the open configuration to the closed configuration. The pivoting contact 6210 can be pivotally coupled to the proximal portion 6202 such that it can accommodate for mechanical perturbations and misalignments when the adapter assembly 6200 is in a closed configuration. Therefore, the pivoting contact 6210 can ensure that proper mechanical contact is established with the thermally conductive surface area 6212 when the adapter assembly 6200 is closed and the sterile barrier is established. Because the pivoting contact 6210 can remain in thermal communication with the thermally conductive surface area 6212, a thermal path is established by which thermal energy can be removed from the internal cavity 6209 of the adapter assembly 6200. Thus, even though contaminants cannot enter the internal cavity 6209 of the adapter assembly 6200, thermal energy can escape the internal cavity 6209 of the adapter assembly 6200 via the pivoting contact of 6210.

According to the non-limiting aspect of FIG. 8, the thermally conductive surface area 6212 can facilitate a convection of the thermal energy from the internal cavity 6209 to the environment of the operating room. Thus, thermal energy can be convected out of the internal cavity 6209 and away from the adapter assembly 6200. Although the non-limiting aspect of FIG. 8 depicts a pivoting contact 6210 with a flat surface area, it shall be appreciated that in other non-limiting aspects, the thermally conductive surface area 6212 can include any number of additional geometric components configured to enhance the amount of heat convected off and away from the adapter assembly 6200. For example, according to some non-limiting aspects, the thermally conductive surface area 6212 further includes a heat sink. Additionally and/or alternatively, the adapter assembly 6200 can include additional heat mitigation channels, baffles, etc., to supplement the removal of thermal energy from the internal cavity 6209.

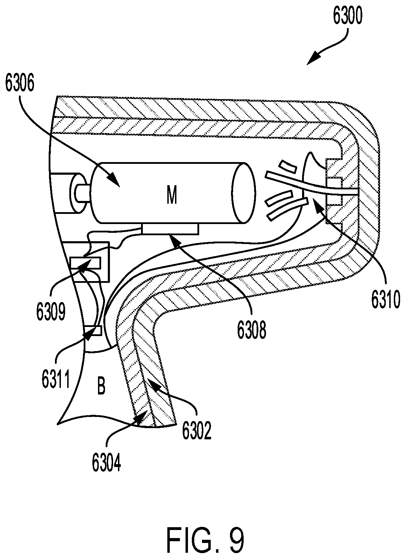

Referring now to FIG. 9 a sectioned side view of a surgical instrument 6300 that includes energy management components 6308, 6310 is depicted in accordance with at least one non-limiting aspect of the present disclosure. According to the non-limiting aspect of FIG. 9, the surgical instrument 6300 can include an adapter assembly 6302 configured to encase a handheld device 6304. The handheld device 6304 can include a motor 6306, which, when in operation, can produce energy. For example, the motor 6306 can produce thermal energy, which can heat up an internal cavity of the adapter assembly 6302. Accordingly, the surgical instrument 6300 can further include a control circuit 6309 coupled to energy management components 6308, 6310 configured to manage the thermal energy produced by the motor 6306.

In further reference to FIG. 9, the surgical device 6300 can include a temperature sensor 6308 configured to generate a signal associated with a temperature of the handheld device 6304 and a piezoelectric oscillating fan 6310. As previously discussed, temperature detection is part of preventative reliability. For example, the surgical instrument could risk overheating since the handheld device 6304-and specifically, the motor 6306-are encased within the sterile barrier established by the adapter assembly 6302. Although this risk can arise from specific external factors, such as a harsh operating environment, the non-limiting aspect of FIG. 9 is configured to monitor the self-heating of electronics within the adapter assembly 6302. By detecting when overheating occurs, the surgical instrument 6300-or an operating clinician-can take preventative action. Accordingly, the temperature sensor 6308 can be specifically configured to function over the expected operating temperature range for the surgical instrument, including a conservative factor of safety.

Still referring to FIG. 9, the temperature sensor 6308 (e.g., a thermocouple, a thermistor, a resistance temperature detector, a semiconductor-based sensor) can generate a signal associated with a temperature of the handheld device 6304. The surgical instrument can further include a control circuit 6309 coupled to the temperature sensor 6308 and configured to receive the signal and determine a temperature of the handheld device 6304 based, at least in part, on the signal generated by the temperature sensor 6308. The control circuit 6309 can also be coupled to a power source 6311 and the piezoelectric oscillating fan 6310. According to some non-limiting aspects, the control circuit 6309 can be positioned within the surgical instrument 6300 itself. Alternatively, the control circuit 6309 can be positioned within the adapter assembly 6302 or a hub to which the surgical instrument 6300 is connected. Regardless of the specific configuration, it shall be appreciated that the temperature sensor 6308 can be implemented with the control circuit 6309 to monitor the temperature of the motor 6306, the handheld device 6304, the adapter assembly 6302, or any other aspect of the surgical instrument 6300 depicted in FIG. 9.

The surgical instrument 6300 of FIG. 9 further includes a piezoelectric oscillating fan 6310 coupled to the control circuit 6309, wherein the piezoelectric oscillating fan 6310 is configured to alter the temperature within the handheld device 6304. For example, if the control circuit 6309 receives a signal from the temperature sensor 6308 and determines that the temperature within the handheld device 6304 has exceeded a predetermined threshold, the control circuit can direct power from the power source 6311 to the piezoelectric oscillating fan 6310, which is configured to lower the temperature within the handheld device 6304 when powered on. Alternatively, the control circuit 6309 can be configured to automatically activate the piezoelectric oscillating fan 6310 whenever the motor 6306 is activated, and to attenuate an operating mode of the piezoelectric oscillating fan 6310 when the temperature exceeds a predetermined threshold. Although the non-limiting aspect of FIG. 9 depicts a piezoelectric oscillating fan 6310, the present disclosure contemplates other non-limiting aspects wherein the surgical instrument utilizes any number of components configured to alter the temperature within the adapter assembly 6302 (e.g., electric fans, cooling plates, heat pipes, synthetic jet air components, electrostatic fluid accelerators). Regardless of the specific combination or method of operation, it shall be appreciated that the combination of the temperature sensor 6308, the control circuit 6309, the power source 6311, and the piezoelectric oscillating fan 6310 can be implemented to manage energy within the adapter assembly 6302, as it is produced by the motor 6306 of the handheld device 6304.

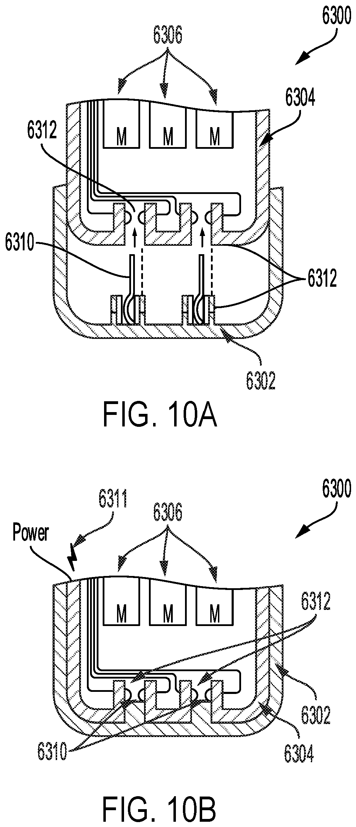

Referring now to FIGS. 10A and 10B, sectioned top views of the surgical instrument 6300 of FIG. 9 are depicted in accordance with at least one non-limiting aspect of the present disclosure. According to the non-limiting aspect of FIGS. 10A and 10B, the adapter assembly 6302 of the surgical device 6300 can include two piezoelectric oscillating fans 6310, which are specifically oriented to be inserted into two corresponding electrical contacts 6312 of the handheld device 6304. When the handheld device 6304 is installed into the adapter assembly 6302, as is depicted in FIG. 10B, the piezoelectric oscillating fans 6310 are received by the electrical contacts 6312 of the handheld device 6304, as is depicted in FIG. 10B. Accordingly, the piezoelectric oscillating fans 6310 are placed in electrical contact with the power source 6311 and/or the control circuit 6309, as depicted in FIG. 9. When the handheld device 6304 is installed in the adapter assembly 6302, the piezoelectric oscillating fans 6310 are further positioned within an inner housing of the handheld device 6304 and thus, can cool the motors 6306 and, generally, the entire interior cavity of the handheld device 6304. As such, the piezoelectric oscillating fans 6310 of FIG. 10B can be activated, receive signals from the temperature sensor 6308 (FIG. 9) and, subsequently, alter an operating temperature of the handheld device 6304 and its components.

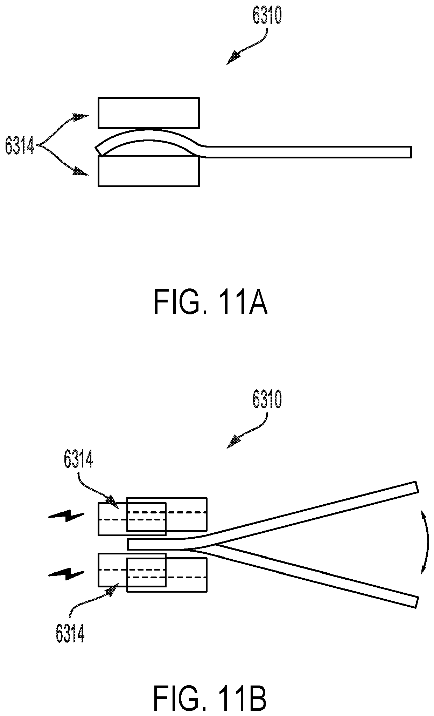

Referring now to FIGS. 11A and 11B, top views of an energy management component 6310 of the adapter assembly 6302 of FIGS. 9 and 10 are depicted in accordance with at least one aspect of the present disclosure. According to the non-limiting aspect of FIGS. 11A and 11B, the piezoelectric oscillating fans 6310 can include electrical contacts 6314 that correspond to the electrical contacts 6312 of the handheld device 6304. In the non-limiting aspect of FIG. 11A, the piezoelectric oscillating fans 6310 are deactivated. In otherwords, the electrical contacts 6314 of the piezoelectric oscillating fans 6310 do not have access to the power source 6311 (FIG. 9). The power source 6311 (FIG. 9) is either turned off or the handheld device 6304 is not properly installed within the adaptor assembly 6302, as is depicted in FIG. 10A. However, in the non-limiting aspect of FIG. 11B, electrical contacts 6314 are energized and the piezoelectric oscillating fans 6310 are oscillating and, therefore, cooling the handheld device 6304 and its internal components (e.g., motors 6306, power source 6311, temperature sensor 6308, and/or control circuit 6309, depicted in FIG. 9). The configuration of FIG. 11B provides an example of the piezoelectric oscillating fans 6310 depicted in FIG. 10B.

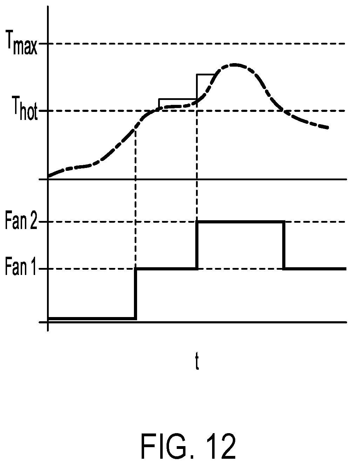

Referring now to FIG. 12, a chart depicting a variable rate of energy management implemented by the surgical instrument 6300 of FIGS. 9-11 is depicted, in accordance with at least one non-limiting aspect of the present disclosure. According to the non-limiting aspect of FIG. 9, the piezoelectric oscillating fans 6310 (FIGS. 9-11) can be configured to oscillate at a variable rate, depending on the temperature detected by the temperature sensor 6308 (FIG. 9). For example, the control circuit 6309 (FIG. 9) can activate a first piezoelectric oscillating fan 6310 when the temperature sensor 6308 (FIG. B9) detects an operating temperature of the handheld device 6304 (FIGS. 9 and 10) has exceeded a first temperature threshold Tₕₒₜ. However, if the temperature does not decrease and instead, continues to exceed a second temperature threshold Tₘₐₓ, the control circuit 6309 (FIG. 9) can activate a second piezoelectric oscillating fan 6310. According to the non-limiting aspect of FIG. 12, the activation of the second piezoelectric oscillating fan 6310 begins to reduce the operating temperature of the handheld device 6304 (FIGS. 9 and 10). Accordingly, the chart of FIG. 12 illustrates a step function indicating a step that correlates to the activation of each piezoelectric oscillating fan 6310, as well as a steady increase and subsequent decrease in the operating temperature of the handheld device 6304 (FIGS. 9 and 10) from Tₕₒₜ to Tₘₐₓ and down once again. The reserve of resources based on the sensed operating temperature of the handheld device 6304 (FIGS. 9 and 10) can result in a more efficient surgical instrument that conserves power and thus, provides an extended life while retaining the energy management benefits discussed in association with FIGS. 9-11.

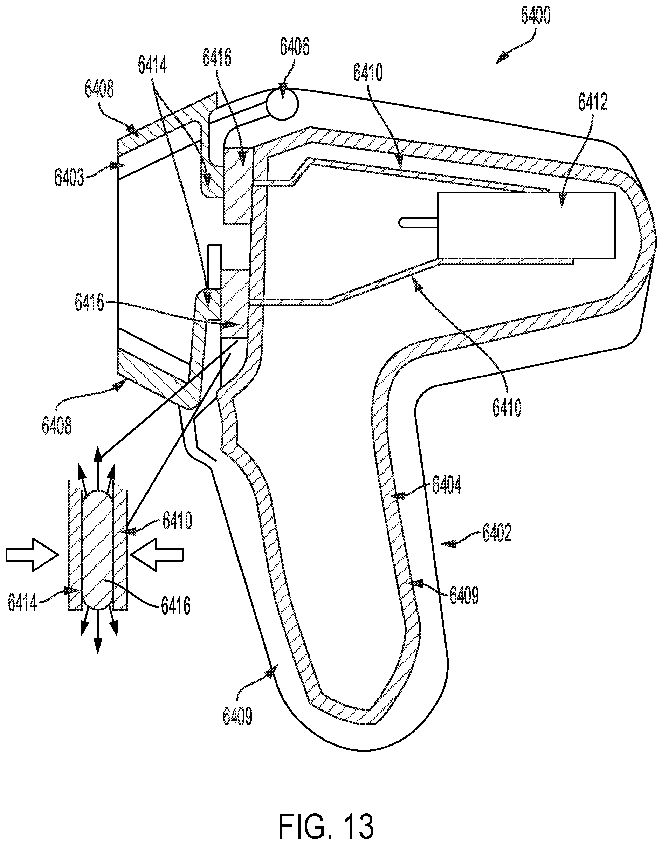

Referring now to FIG. 13, a sectioned side view of a surgical instrument including a handheld surgical device 6404 and an adapter assembly 6400 that includes energy management components 6408, 6410, 6414 is depicted in accordance with at least one non-limiting aspect of the present disclosure. According to the non-limiting aspect of FIG. 13, the adapter assembly 6400 can include proximal portion 6402 and a distal portion 6403 connected in a clamshell configuration via a hinge 6406. Collectively, the proximal portion 6402 and the distal portion 6403 can constitute an outer shell or housing that defines an internal cavity 6409 configured to encase a handheld device 6404 with a motor 6412 that can generate energy when the surgical instrument is in use. Accordingly, the adapter assembly 6400 can be configured to transition from an open configuration-wherein the hinge 6206 is open and the sterile barrier is disrupted-to a closed configuration, wherein the hinge 6406 is closed and the sterile barrier is established. Collectively, the proximal portion 6402 and the distal portion 6403 can define a handle portion configured for the ergonomic handling of the surgical instrument when the handheld device 6404 is installed within the adapter assembly 6400. As can be seen in FIG. 13, the proximal portion 6402 and the distal portion 6403 of the adapter assembly 6400 can each include energy management components 6408, 6410, 6414 configured to effectively manage energy created by a the motor 6412 when the handheld device 6404 is installed within an internal cavity 6409 of the adapter assembly 6400.

Still referring to FIG. 13, the adapter assembly 6400 can include a heat sink assembly including an external heat sink 6408 and an internal heat sink 6410 positioned within the internal cavity 6409 of the adapter assembly 6400. According to the non-limiting aspect of FIG. 13, the external heat sink 6408 is positioned on an external surface of the distal portion 6403 of the adapter assembly 6400 and is configured to convect thermal energy produced by the motor 6412 away from the adapter assembly 6400. In some non-limiting aspect, the external heat sink can include a plurality of fins configured to expand the surface area off of which thermal energy can be convected. The internal heat sink 6410 can be positioned within the proximal portion 6402 of the adapter assembly 6400 and configured to mechanically contact a motor 6412 of the handheld device 6404, thereby creating a conductive thermal path for thermal energy to be routed off of—and away from—the motor 6412. A second internal heat sink 6414 can be positioned within the distal portion 6403 of the adapter assembly 6400 and configured to mechanically contact the external heat sink 6408 while preserving the sterile barrier formed by the adapter assembly 6400. According to the non-limiting aspect of FIG. 13, the external heat sink 6408 can include an internal portion 6414 configured to traverse inside the internal cavity 6409 of the adapter assembly 6400 while maintaining the sterile barrier when the adapter assembly 6400 is in its closed configuration. The internal portion 6414 of the external heat sink 6408 can be further configured to mechanically contact a compressible, conductive material 6416. The compressible, conductive material 6416 can be configured to interface the internal heat sink 6410 and the internal portion 6414 of the external heat sink 6408 when the hinge 6406 is closed, thereby extending the thermally conductive path from the motor 6412 to the external heat sink 6408, where it can be convected away from the surgical instrument. Accordingly, when the hinge 6406 is closed and the surgical instrument is being used by a clinician, the heat sink assembly can transfer thermal energy generated by the motor 6412 away from the internal cavity 6409.

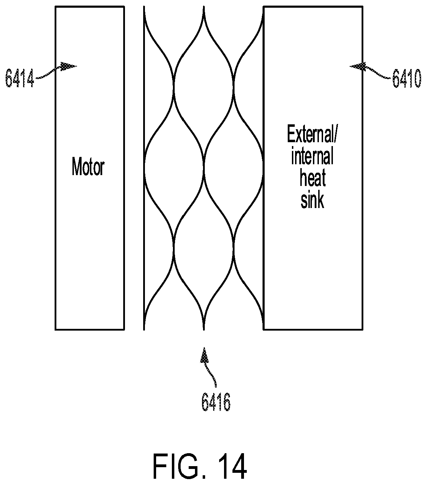

Referring now to FIG. 14, the compressible, conductive material 6416 of FIG. 13 is depicted in accordance with at least one non-limiting aspect of the present disclosure. According to the non-limiting aspect of FIG. 14, the compressible, conductive material 6416 can be configured to compress, thereby establishing an interference fit between the internal heat sink 6410 and the internal portion 6414 of the external heat sink 6408. According to the non-limiting aspect of FIG. 13, the compressible, conductive material 6416 can improve the conductive efficiency between the internal heat sink 6410 and the internal portion 6414 of the external heat sink 6408. For example, the compressible, conductive material 6416 can include a metal mesh (e.g., scouring, sponge, type material) or a thermally conductive elastomer, among others. The compressible, conductive material 6416 of FIG. 14 can be configured to compress around imperfections, thereby filling discontinuities in the collective, conductive thermal path established by the internal heat sink 6410 and the internal portion 6414 of the external heat sink 6408. Accordingly, the compressible, conductive material 6416 can account for thermal and dimensional tolerances.

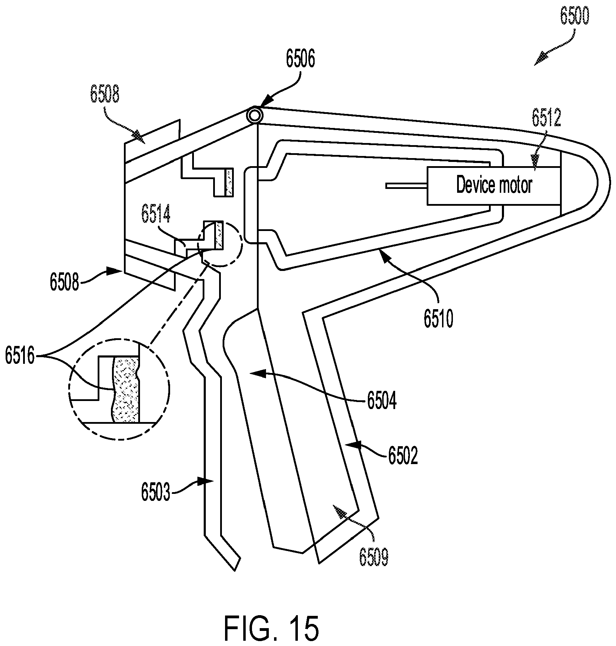

Referring now to FIG. 15, a sectioned side view of a surgical instrument including a handheld surgical device 6504 and an adapter assembly 6500 that includes energy management components 6508, 6510, 6514, 6516 is depicted in accordance with at least one non-limiting aspect of the present disclosure. According to the non-limiting aspect of FIG. 15, the adapter assembly 6500 can include proximal portion 6502 and a distal portion 6503 connected in a clamshell configuration via a hinge 6506. Collectively, the proximal portion 6502 and the distal portion 6503 can constitute an outer shell or housing that defines an internal cavity 6509 configured to encase a handheld device 6504 that can generate energy when the surgical instrument is in use. Accordingly, the adapter assembly 6500 is configured to transition from an open configuration, wherein the hinge 6206 is open and the sterile barrier is disrupted, to a closed configuration, wherein the hinge 6506 is closed and the sterile barrier is established. Collectively, the proximal portion 6502 and the distal portion 6503 can define a handle portion configured for the ergonomic handling of the surgical instrument when the handheld device 6504 is installed within the adapter assembly 6500. As can be seen in FIG. 15, the proximal portion 6502 and distal portion 6503 of the adapter assembly 6500 can each include energy management components 6508, 6510, 6514, 6516 configured to effectively manage energy created by a handheld device when installed within an internal cavity 6509 of the adapter assembly 6500.

Still referring to FIG. 15, the adapter assembly 6500 can include a heat sink assembly including an external heat sink 6508 and several internal heat sinks 6510, 6514 positioned within the internal cavity 6509 of the adapter assembly 6500. According to the non-limiting aspect of FIG. 15, the external heat sink 6508 is positioned on an external surface of the distal portion 6503 of the adapter assembly 6500 and is configured to convect thermal energy produced by the motor 6512 away from the surgical instrument 6500. In some non-limiting aspect, the external heat sink can include a plurality of fins configured to expand the surface area off of which thermal energy can be convected. A first internal heat sink 6510 can be positioned within the proximal portion 6502 of the adapter assembly 6500 and configured to mechanically contact a motor 6512 of the handheld device 6504, thereby creating a conductive thermal path for thermal energy to be routed off of—and away from—the motor 6512. A second internal heat sink 6514 can be positioned within the distal portion 6503 of the adapter assembly 6500 and configured to mechanically contact the external heat sink 6508 while preserving the sterile barrier formed by the adapter assembly 6500. The second internal heat sink 6514 can be further configured to mechanically contact with the first heat sink 6510 when the hinge 6506 is closed, thereby extending the thermally conductive path from the motor 6512 to the external heat sink 6508. Accordingly, when the hinge 6506 is closed and the surgical instrument is being used by a clinician, the heat sink assembly can transfer thermal energy generated by the motor 6512 away from the internal cavity 6509. The non-limiting aspect of FIG. 15 is notably depicted in an open configuration, and thus, the second heat sink 6514 is not depicted in mechanical contact with the first heat sink 6510.

In further reference to FIG. 15, the heat sink assembly can further include a thermal paste 6516 (e.g., thermal compound, grease) configured to interface the first internal heat sink 6510 and the second internal heat sink 6514. According to the non-limiting aspect of FIG. 15, the thermal paste 6516 can improve the conductive efficiency between the first internal heat sink 6510 and the second internal heat sink 6514 and, thus, the external heat sink 6508. Additionally, the thermal paste 6516 can be configured to alleviate hot spots that typically develop between coupled heat sinks by filling discontinuities in the collective, conductive thermal path established by the first internal heat sink 6510 and second internal heat sink 6514 and accounting for thermal and dimensional tolerances. The thermal paste 6516 of FIG. 15 can be similar to those used in integrated circuit electronics, as are typically applied between computer processing units and corresponding heat sinks. For example, although the thermal paste 6516 can be thermally conductive, it may not be electrically conductive, thereby reducing the potential for shocks and/or short circuits. The thermal paste 6516 can be pre-applied to the adapter assembly 6500 and re-applied to the handheld device 6504 when the adapter assembly 6500-and sterile barrier-needs to be replaced. The thermal paste 6516 can offer several advantages over graphite pads and/or thermally conductive pads, which can break down over time and, thus, become less efficient. Additionally, the thermal paste 6516 contemplated by the present disclosure is less expensive than comparable graphite and/or thermally conductive pads.

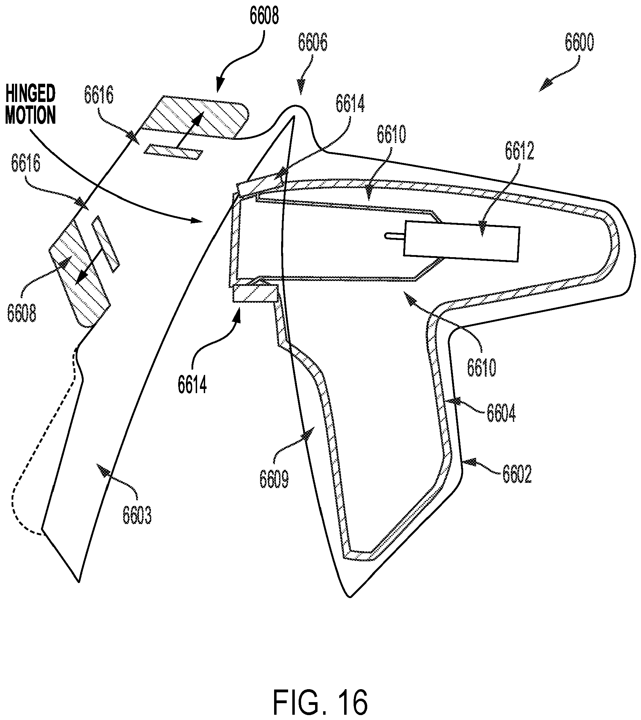

Referring now to FIG. 16, a sectioned side view of a surgical instrument including a handheld surgical device 6604 and an adapter assembly 6600 that includes energy management components 6608, 6610, 6614, 6616 is depicted in accordance with at least one non-limiting aspect of the present disclosure. According to the non-limiting aspect of FIG. 16, the adapter assembly 6600 can include a proximal portion 6602 and a distal portion 6603 connected in a clamshell configuration via a hinge 6606. Collectively, the proximal portion 6602 and the distal portion 6603 can constitute an outer shell or housing that defines an internal cavity 6609 configured to encase a handheld device 6604 that can generate energy when the surgical instrument is in use. Accordingly, the adapter assembly 6600 is configured to transition from an open configuration, wherein the hinge 6606 is open and the sterile barrier is disrupted, to a closed configuration, wherein the hinge 6606 is closed and the sterile barrier is established. Collectively, the proximal portion 6602 and the distal portion 6603 can define a handle portion configured for the ergonomic handling of the surgical instrument when the handheld device 6604 is installed within the adapter assembly 6600. As can be seen in FIG. 16, the proximal portion 6602 and distal portion 6603 of the adapter assembly 6600 can each include energy management components 6608, 6610, 6614, 6616 configured to effectively manage energy created by a handheld device when installed within an internal cavity 6609 of the adapter assembly 6600.

Still referring to FIG. 16, the adapter assembly 6600 can include a heat sink assembly including an external heat sink 6608 and several internal heat sinks 6610, 6614, 6616 positioned within the internal cavity 6609 of the adapter assembly 6600. According to the non-limiting aspect of FIG. 16, the external heat sink 6608 is positioned on an external surface of the distal portion 6603 of the adapter assembly 6600 and is configured to convect thermal energy produced by the motor 6612 away from the surgical instrument 6600. In some non-limiting aspect, the external heat sink 6608 can include a plurality of fins configured to expand the surface area off of which thermal energy can be convected. An internal heat sink 6610 can be positioned within the proximal portion 6602 of the adapter assembly 6600 and configured to mechanically contact a motor 6612 of the handheld device 6604, thereby creating a conductive thermal path for thermal energy to be routed off of—and away from—the motor 6612. The internal heat sink 6610 can terminate in a wedge-shaped, thermally conductive surface area 6614. The thermally conductive surface area 6614 can be configured to mechanically contact a translating conductor 6616 positioned within the distal portion 6603 of the adapter assembly 6600. The translating conductor 6616 can be further configured to move between a first position and a second position within the distal portion 6603 of the adapter assembly 6600. For example, when the adapter assembly 6600 is in the closed configuration, the translating conductor 6616 makes mechanical contact with the thermally conductive surface area 6614, which is moved from the first position to the second position based, at least in part, on the wedge-shaped configuration of the thermally conductive surface area 6614. In the second position, the translating conductor 6616 is in mechanical contact with the external heat sink 6608, thereby extending the thermally conductive path from the motor 6612 to the external heat sink 6608 while preserving the sterile barrier formed by the adapter assembly 6600.

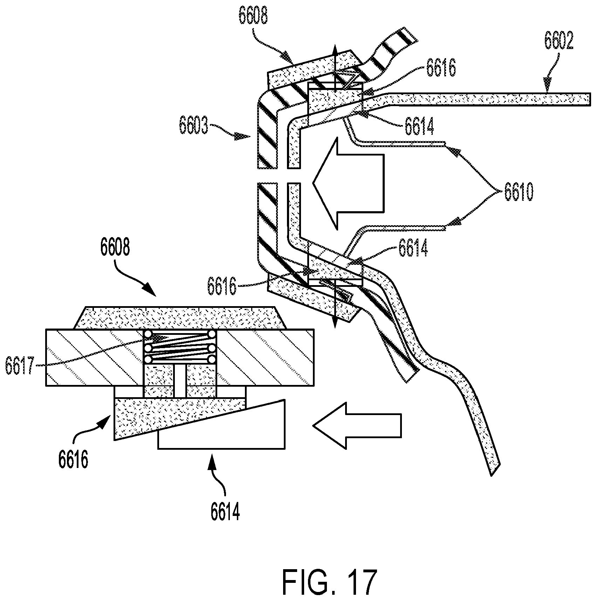

Referring now to FIG. 17, a sectioned side view of the energy management components 6608, 6610, 6614, 6616 of FIG. 16 is depicted in accordance with at least one non-limiting aspect of the present disclosure. According to the non-limiting aspect of FIG. 17, the internal heat sink 6610 can mechanically contact the motor 6612 of the handheld device 6604 (FIG. 16) and can terminate in a wedge-shaped, thermally conductive surface area 6614. The translating conductor 6616 is further illustrated as configured with a corresponding geometry to the wedge shape of the thermally conductive surface area 6414. As is depicted in FIG. 17, as the proximal portion 6602 of the adapter assembly 6600 (FIG. 16) moves towards the distal portion 6603 of the adapter assembly 6600, the wedge-shaped, thermally conductive surface area 6614 forces the translating conductor 6616 up towards the external heat sink 6608. The non-limiting aspect of FIG. 17 further includes a spring 6617, which can be configured to movably couple the translating conductor 6616 to an interior wall of the internal cavity 6609 (FIG. 16) or in some aspects, to the external heat sink 6608 itself. Although the non-limiting aspect of FIG. 17 includes a wedge-shaped geometry, it shall be appreciated that any corresponding geometry capable of engaging the thermally conductive surface area 6614 and thus, moving the translating conductor 6616 into mechanical contact with the external heat sink 6608 can be employed to extend the thermally conductive path from the motor 6612.

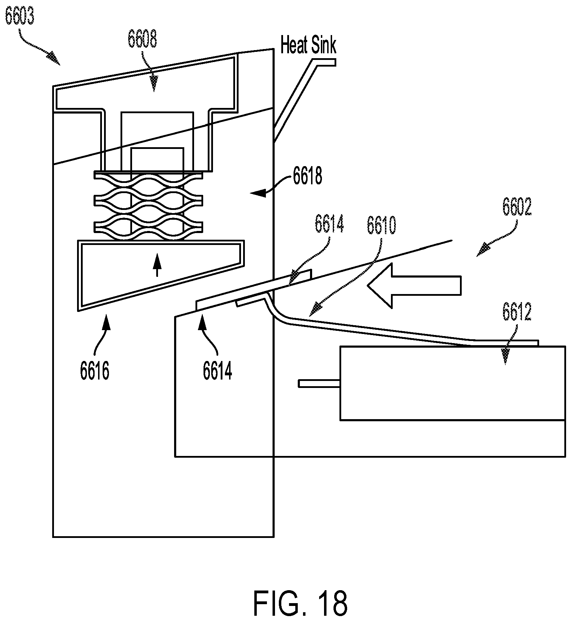

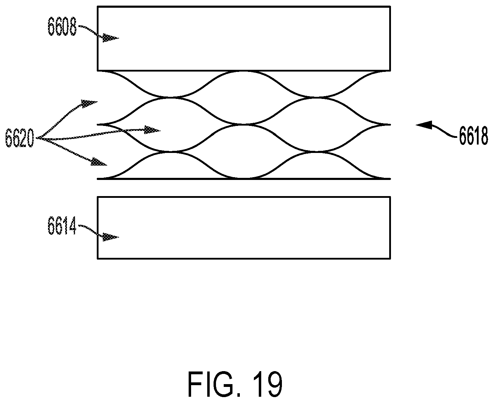

Referring now to FIGS. 18 and 19, the adapter assembly 6600 of FIG. 16 is depicted in accordance with another non-limiting aspect of the present disclosure. According to the non-limiting aspect of FIGS. 18 and 19, the spring 6617 of FIG. 17 includes a wave spring 6618 geometry that is dispositioned within the distal portion 6603 between the translating conductor 6616 and the wedge-shaped, thermally conductive surface area 6614. The wave spring 6618 can include any compressible and/or elastic material that is thermally conductive, rendering it suitable for efficiently transferring thermal energy from the translating conductor 6616 to the external heat sink 6608. As is depicted in FIG. 19, the wave spring 6618 can include a plurality of internal pockets 6620 that provide the spring 6617 with an increased surface area. When compressed, the pockets 6620 can compress, causing the interior walls of the pockets 6620 to contact one another. Accordingly, the wave spring 6618-and more specifically, the pockets 6620-can increase the conductive surface area of the thermal path, thereby creating a more efficient removal of thermal energy from the internal cavity 6609 of the adapter assembly 6000. Although the wave spring 6618 of FIGS. 18 and 19 include a specific geometry, it shall be appreciated that any geometry configured to enable the movement of the translating conductor 6616 relative to the external heat sink 6608 while increasing the conductivity of the thermal path to the motor 6612 can be implemented to achieve an improved efficiency of heat transfer.

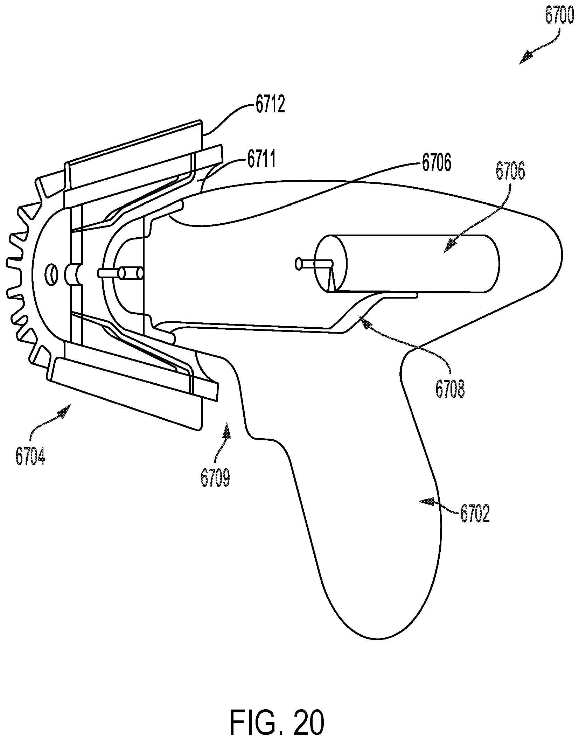

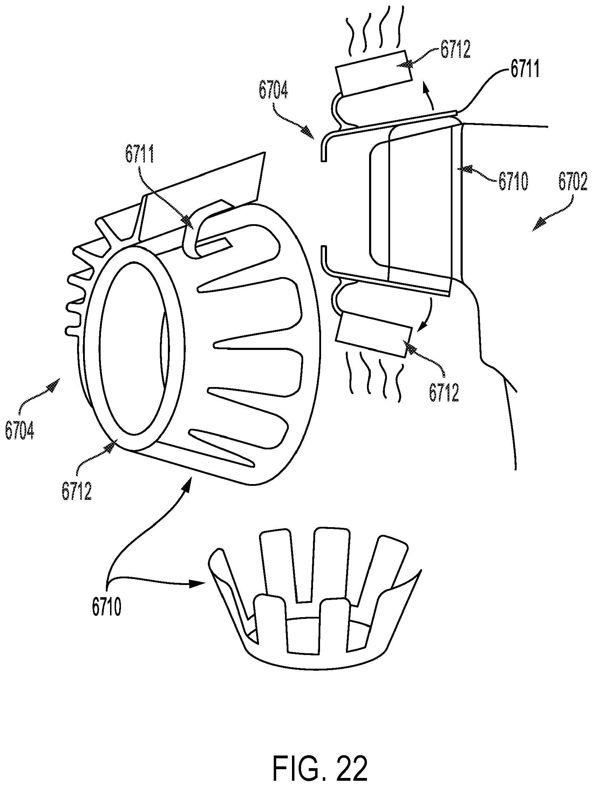

Referring now to FIG. 20, a sectioned perspective view of a surgical instrument 6700 including a handheld surgical device 6702 and a distal portion 6704 of an adapter assembly with energy management components 6708, 6710 (FIG. 22), 6712 is depicted in accordance with at least one non-limiting aspect of the present disclosure. According to the non-limiting aspect of FIG. 20, the distal portion 6704 of the adapter assembly includes an external heat sink 6712. In some non-limiting aspects, the distal portion 6704 can be connected to a proximal portion of the adapter assembly in a clamshell configuration via a hinge. Regardless, the distal portion 6704 of the adapter assembly partially defines an outer shell that includes an internal cavity 6709 configured to encase a handheld device 6702. Similar to other disclosed aspects, a motor 6706 of the handheld device 6702 can generate energy when the surgical instrument 6700 is in use. Accordingly, the distal portion 6704 can be configured to mechanically couple to the handheld device 6702, thereby establishing a sterile barrier. As can be seen in FIG. 20, the distal portion 6704 of the adapter assembly and the handheld device 6702 can collectively include energy management components 6708, 6710 (FIG. 22), 6712 configured to effectively manage energy created by a handheld device 6702 when installed within an internal cavity 6709 of the adapter assembly.

In further reference to FIG. 20, the surgical instrument 6700 can include an external heat sink 6712 and several internal heat sinks 6708, 6710 (FIG. 22), 6711 positioned within the internal cavity 6709 of the adapter assembly. According to the non-limiting aspect of FIG. 20, the external heat sink 6712 can be positioned on an external surface of the distal portion 6704 of the adapter assembly and is configured to convect thermal energy produced by the motor 6706 away from the surgical instrument 6700. In some non-limiting aspects, the external heat sink 6712 can include a plurality of fins configured to expand the surface area off of which thermal energy can be convected. An internal heat sink 6708 can be positioned within the internal cavity 6709 of the adapter assembly and configured to mechanically contact the a motor 6706 of the handheld device 6702, thereby creating a conductive thermal path for thermal energy to be routed off of—and away from—the motor 6706. The internal heat sink 6708 can terminate in thermally conductive surface area 6710 positioned proximal to a distal end of the handheld device 6702. The thermally conductive surface area 6710 can be configured to mechanically contact a leaf spring 6711 coupled to an internal surface of the external heat sink 6712 when the handheld device 6702 is properly installed within the internal cavity 6709 and arranged within the distal portion 6704 of the adapter assembly.

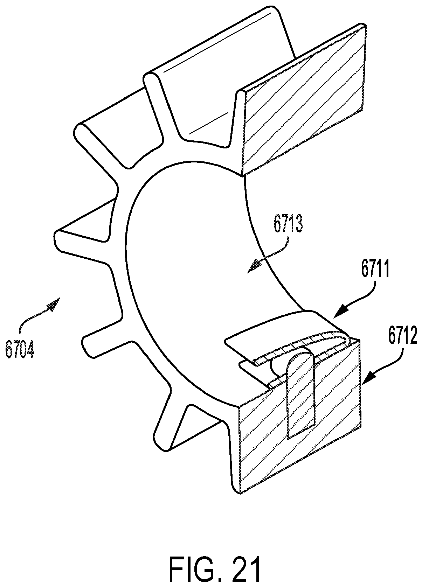

Referring now to FIG. 21, a sectioned perspective view of the energy management components 6710 (FIG. 22), 6711, 6712 of FIG. 20 is depicted in accordance with at least one non-limiting aspect of the present disclosure. According to the non-limiting aspect of FIGS. 20 and 21, the leaf spring 6711 is coupled to an internal surface 6713 of the external heat sink 6712. Specifically, the mechanical nature of the leaf spring 6711 is depicted in FIG. 21. For example, it shall be appreciated that the leaf spring 6711 can include a specific elastic nature, enabling it to apply an inward force when compressed. Accordingly, when the handheld device 6702 (FIG. 20) is properly installed within the internal cavity 6709 (FIG. 20) and arranged within the distal portion 6704 of the adapter assembly, the leaf spring 6711 applies an inward force on the thermally conductive surface area 6710 (FIG. 22). This ensures that the leaf spring 6711 remains in mechanical engagement with the thermally conductive surface area 6710, thereby establishing a conductive path capable of efficiently removing thermal energy from the motor 6706 of the surgical instrument 6700. The leaf spring 6711 can be either integrally formed with the thermally conductive surface area 6710 or attached separately. In other non-limiting aspects, the leaf spring 6711 can be mechanically coupled to the thermally conductive surface area 6710 and configured to mechanically contact an internal surface of the external heat sink 6712 when the handheld device 6702 is properly installed within the internal cavity 6709 and arranged within the distal portion 6704 of the adapter assembly.

Referring now to FIG. 22, various views of the energy management components 6710, 6711, 6712 of FIGS. 20 and 21 are depicted in accordance with at least one non-limiting aspect of the present disclosure. According to the non-limiting aspect of FIG. 22, the leaf spring 6711 can be positioned between the thermally conductive surface area 6710 of the handheld device 6702 (FIG. 20) and compressed when the handheld device 6702 is properly installed within the internal cavity 6709 (FIG. 20) and arranged within the distal portion 6704 of the adapter assembly. Due to the corresponding frustoconical structure of the thermally conductive surface area 6710 and distal portion 6704 of the adapter assembly, the leaf spring 6711 can compress more and more as the handheld device 6702 is installed. Due to the elastic nature of the leaf spring 6711, the inward force applied by the leaf spring 6711 gradually increases, thereby increasing the surface area by which the thermally conductive surface area 6710, the leaf spring 6711, and the external heat sink 6712 are in thermally conductive contact. It shall be appreciated that conductive efficiency improves as the conductive surface area increases. Therefore, the energy management components 6710, 6711, 6712 of FIGS. 20 and 21 can be implemented to effectively remove thermal energy generated by the motor 7606 (FIG. 20) from the internal cavity 6709 (FIG. 20) of the surgical instrument 6700 (FIG. 20).

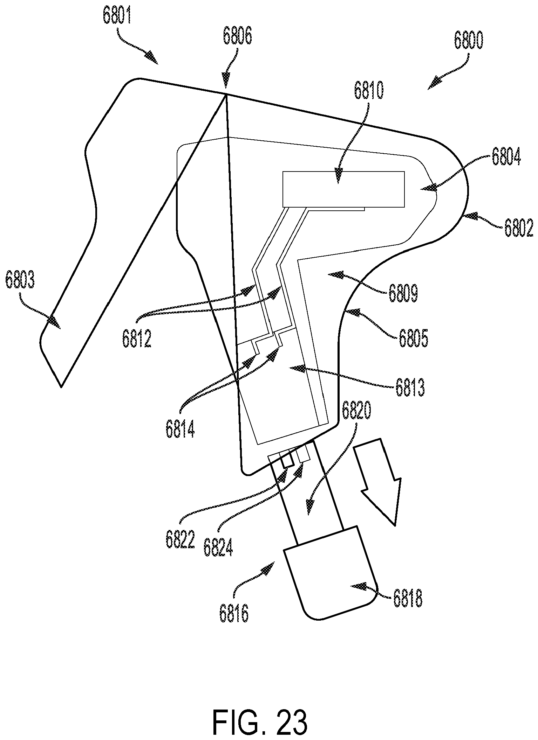

Referring now to FIG. 23, a sectioned side view of a surgical instrument 6800 including a handheld surgical device 6804 and an adapter assembly 6801 that includes energy management system 6610, 6614, 6616 is depicted in accordance with at least one non-limiting aspect of the present disclosure. According to the non-limiting aspect of FIG. 23, the surgical instrument 6800 can include a proximal portion 6802 and a distal portion 6803 connected in a clamshell configuration via a hinge 6806. Collectively, the proximal portion 6802 and the distal portion 6803 can constitute an outer shell or housing that defines an internal cavity 6809 configured to encase the handheld device 6804 configured to generate energy when the surgical instrument is in use. Accordingly, the adapter assembly 6801 can be configured to transition from an open configuration, wherein the hinge 6806 is open and the sterile barrier is disrupted, to a closed configuration, wherein the hinge 6806 is closed and the sterile barrier is established. Collectively, the proximal portion 6802 and the distal portion 6603 can further define a handle portion 6805 configured for the ergonomic handling of the surgical instrument 6800 when the handheld device 6804 is installed within the adapter assembly 6801. As can be seen in FIG. 23, the proximal portion 6802 and distal portion 6803 of the adapter assembly 6800 can each include energy management components 6812, 6814, 6813, 6816 configured to effectively manage energy created by a handheld device 6804 when installed within an internal cavity 6809 of the adapter assembly 6801.

Still referring to FIG. 23, the surgical instrument 6800 can include an energy storage and removal assembly 6812, 6814, 6813, 6816 including a removable thermal energy storage device 6816 configured to be installed within a dedicated compartment 6813 within the internal cavity 6809 of the adapter assembly 6801. According to the non-limiting aspect of FIG. 23, one or more internal heat sinks 6812 can be configured to mechanically contact a motor 6810 of the handheld device 6804, thereby creating a conductive thermal path for thermal energy to be routed off of—and away from—the motor 6810. The one or more internal heat sinks 6812 can terminate in one or more conductive contacts 6814 positioned within the dedicated compartment 6813 of the internal cavity 6809. When properly installed within the dedicated compartment 6813, the removable thermal energy storage device 6816 can be configured to mechanically contact the one or more contacts 6814, thereby extending the thermally conductive path into the removable thermal energy storage device 6816.

In further reference to FIG. 23, rather than utilizing an external heat sink configured to convect and/or radiate heat away from the handheld device 6804, the surgical instrument 6800 can route thermal energy away from the motor 6810 and store it within the thermal energy storage device 6816. For example, the thermal energy storage device 6816 can include a material including a high specific heat configured to dissipate heat throughout the storage device 6816 and strategically retard any rise in internal temperature. The removable storage device 6816 can include one or more conductive contacts 6824 configured to engage the conductive contacts 6814 positioned within the dedicated compartment when the storage device 6816 is properly installed within the adapter assembly 6801. Accordingly, the removable storage device 6816 of FIG. 23 can be configured to charge-that is, receive and store thermal energy generated by the motor 6810-as the surgical device 6800 is in use. Specifically, the material with the high specific heat can absorb and dissipate thermal energy it receives from the motor 6810 throughout the storage device 6816. For example, the material can include a solid ingot or a liquid such as water. Of course, other non-limiting aspects contemplated by the present disclosure contemplate any number of suitable materials for the removable storage device 6816. When the storage device 6816 achieves a critical temperature, it can be removed from the dedicated compartment and replaced with a similarly configured-albeit cooler-storage device 6816. The replacement can either be ambient temperature or pre-cooled below an ambient temperature to further delay the time it takes to achieve a critical temperature.

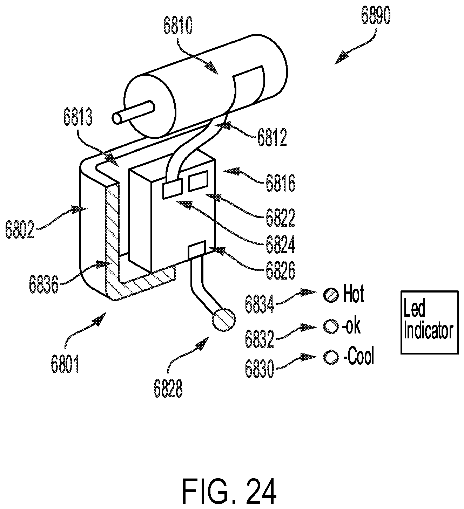

Referring now to FIG. 24, a sectioned perspective view of the energy management components 6824, 6826, 6828 of the energy management system 6816 of FIG. 23 is depicted in accordance with at least one aspect of the present disclosure. According to the non-limiting aspect of FIG. 24, either the dedicated compartment 6813, the storage device 6816, or both can include a temperature sensor 6822 (e.g., a thermocouple, a thermistor, a resistance temperature detector, a semiconductor-based sensor) configured to generate a signal associated with an operating temperature of the removable storage device 6816. The surgical instrument 6800 can further include a control circuit 6826 coupled to the temperature sensor 6822 and configured to receive the signal and determine a temperature of the removable storage device 6816 based, at least in part, on the signal generated by the temperature sensor 6822. Accordingly, the control circuit can determine that the temperature of the removable storage device 6816 has exceeded a predetermined threshold and, thus, notify a clinician via alert.

Still referring to FIG. 24, the energy management system can further include a light emitting diode indicator 6828 coupled to the control circuit 6826 that can be configured to indicate the determined operating temperature of the removable storage device 6816 to a clinician. According to the non-limiting aspect of FIG. 24, the indicator 6828 can illuminate a specific color associated with the operating temperature of the removable storage device 6816. For example, the indicator can illuminate a first color 6830 to indicate that the storage device 6816 is of a cool temperature, a second color 6832 to indicate that the storage device 6816 is of a warm temperature, and a third color 6834 to indicate that the storage device 6816 is of a hot temperature. When the indicator is illuminated the third color 6834, the operating clinician can remove and/or replace the removable storage device 6816. Although the non-limiting aspect of FIG. 24 illustrates a light emitting diode indicator 6828, the present disclosure contemplates other non-limiting aspects featuring a variety of different alerts, including audible, haptic, and/or visual alerts. Likewise, the surgical instrument 6800 of FIG. 23 can be alerted to include any number of user interface components, including screens, speakers, motors, lights, and/or the like. As previously discussed, the storage device 6816 can include a solid ingot. Alternatively, the storage device 6816 can include a hollow cavity and/or bladder comprising a fluid, such as water. Additionally and/or alternatively, the adapter assembly 6801 can include insulation 6836 positioned between an interior wall of the dedicated compartment 6813 to further manage and/or contain any thermal energy generated by the motor 6810 that is not stored within the storage device 6816. Accordingly, the indicator 6828 and removable storage device 6816 of FIGS. 23 and 24 can be utilized to effectively manage energy dissipated by the handheld device 6804, thereby facilitating a safe and continued use of the surgical instrument 6800.

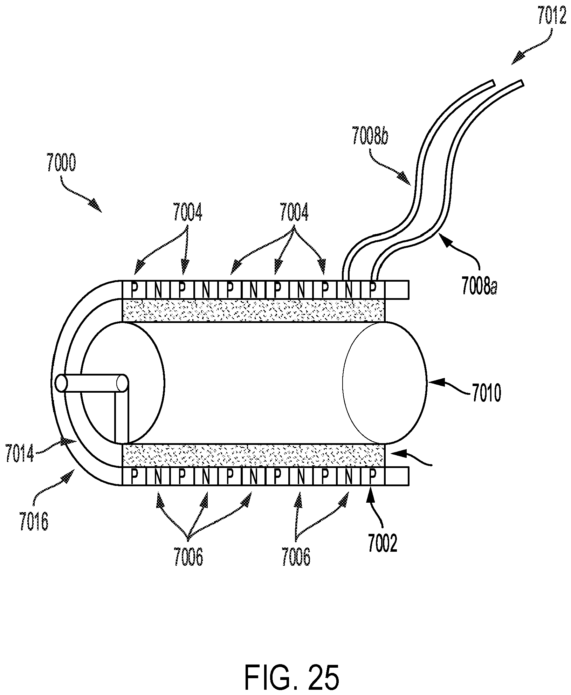

Referring now to FIG. 25, a sectioned perspective view of an energy management system 7000 of a surgical instrument is depicted in accordance with at least one non-limiting aspect of the present disclosure. According to the non-limiting aspect of FIG. 25, the energy management system 7000 can include a thermoelectric cooling configuration, including a Peltier module 7002 configured to assist in the management of thermal energy generated by the motor 7010. The Peltier module 7002 can be configured to utilize a thermoelectric effect, which utilizes an electric current configured to flow between two material junctions, which can cause cooling. In the non-limiting aspect of FIG. 25, the Peltier module 7002 can include a matrix of PIN junctions dispositioned between a plurality of P nodes 7004 and a plurality of N nodes 7006. The plurality of P nodes 7004 and the plurality of N nodes 7006 collectively constitute a matrix of joined electrical conductors 7004, 7006 that can be connected to a power source 7012 via a pair of leads 7008a, 7008b. The power source 7012 can thus apply a voltage across the matrix of joined conductors 7004, 7006 to create an electric current. When the current flows through the junctions of the two conductors 7004, 7006, thermal energy can be removed from a first side 7014 of the matrix of the two conductors 7004, 7006 and deposited on a second side 7016 of the matrix of the two conductors 7004, 7006. The first side 7014 can be configured to abut the motor 7010, and the second side 7016 can be positioned away from motor 7010 such that thermal energy is pulled away from the motor 7010 to prevent overheating. According to some non-limiting aspects, the energy management system 7000 of FIG. 25 can further include a heat sink to assist in dispelling the thermal energy that is pulled away from the motor 7010 via the Peltier module 7002.

Although the non-limiting aspects of FIGS. 5-25 depict energy management systems configured to manage the generation of thermal energy, it shall be appreciated that similar systems can be implemented to effectively manage the generation of a wide variety of energies produced by the motor, handheld device, or surgical instrument as a whole. For example, the following aspects can be implemented to assist with the management of vibrational and/or acoustic energy generated by the motor of a handheld device when the surgical instrument is in use. As is the case with thermal energy, the inclusion of an adapter assembly that establishes a sterile barrier around the handheld device can complicate the dissipation of vibrational and/or acoustic energy. Without a proper means of managing this energy, the surgical instrument can suffer from reduced accuracy and/or an accelerated degradation of components and can become difficult for a clinician to handle. Accordingly, there is a need for energy management systems that can be configured to manage and mitigate the generation of vibrational and/or acoustic energy.

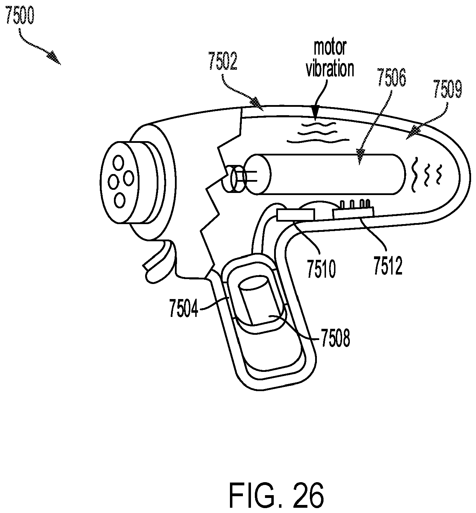

Referring now to FIG. 26, a sectioned perspective view of a surgical instrument 7500 including a handheld surgical device and an adapter assembly 7502 that includes an energy management system 7504 is depicted in accordance with at least one non-limiting aspect of the present disclosure. According to the non-limiting aspect of FIG. 26, the surgical instrument 7500 can include an adapter assembly 7502 with an outer shell housing that defines an internal cavity 7509. A handheld device can be installed within the internal cavity 7509, including its requisite components. For example, the non-limiting aspect of FIG. 26 includes a motor 7506, a power source 7508, a sensor 7510, and a control circuit 7512 within the internal cavity 7509. The motor 7506, specifically, can produce vibrational energy when the surgical instrument 7500 is in operation, as is depicted in FIG. 26.

In further reference to FIG. 26, the surgical instrument can include an energy management system 7504 dispositioned within the internal cavity 7509 of the adapter assembly 7502. According to the non-limiting aspect of FIG 26, the surgical instrument 7500 can include composites 7504, which are strategically situated throughout the internal cavity 7509, wherein the composites 7504 are configured to manage the vibrational energy generated by the motor 7506. For example, the composites 7504 can include piezoelectric characteristics that are configured to dampen the vibrational energy by producing a counterforce to the generated vibrational energy when activated. In some non-limiting aspects, the composites can be configured to automatically produce the aforementioned counterforces as soon as the motor 7506 is activated. As will be discussed, the counterforces can be specifically configured to mitigate and/or substantially eliminate the vibrational energy generated by the motor 7506. For example, the composites 7504 can produce counterforces that are equal, albeit opposite, to the vibrational energy generated by the motor 7506.

According to other non-limiting aspect of FIG. 26, the sensor 7510 can detect the vibrational energy generated by the motor 7506 when the surgical instrument 7500 is in use. The sensor 7510 can generate a signal associated with the detected vibrational energy. The control circuit 7512 can be configured to receive the signal from the sensor 7510 and determine an operational level of the vibrational energy produced by the motor 7506 when the surgical instrument 7500 is in use. Upon determining that the operational level of the vibrational energy produced by the motor 7506 exceeds a predetermined threshold, the control circuit 7512 can route energy from the power source 7508 to the piezoelectric composites 7504. Upon activation, the piezoelectric composites 7504 can be configured to generate the counterforce, thereby dampening the vibrational energy generated by the motor 7506 when the surgical instrument 7500 is in use. Accordingly, the surgical instrument 7500 of FIG. 26 can be configured self-stabilize, making it easier for an operating clinician to use.

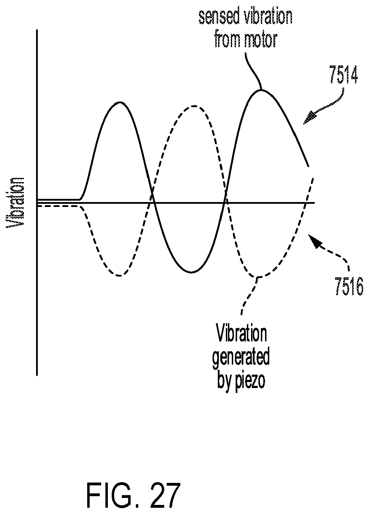

Referring now to FIG. 27, a chart depicting an energy response 7516 of the energy management system 7504 of FIG. 26 is depicted in accordance with at least one non-limiting aspect of the present disclosure. As was previously discussed, the counterforces 7516 produced by the composites 7504 of FIG. 26 can be specifically configured to mitigate and/or substantially eliminate the vibrational energy 7514 generated by the motor 7506 (FIG. 26). According to the non-limiting aspect of FIG. 27, the composites 7504 of FIG. 26 can produce counterforces 7516 that are equal-albeit opposite-to the vibrational energy 7514 generated by the motor 7506. As such, the composites of FIG. 26 can reduce the vibrational energy 7514 generated by the motor 7506 (FIG. 26), improving the stability of the surgical instrument 7500 and, therefore, the accuracy with which an operating clinician can use the surgical instrument 7500. Although the non-limiting aspect of FIG. 27 depicts an energy response 7516 configured to match the vibrational energy generated by the motor 7506 (FIG. 26), it shall be appreciated that the energy management system 7504 contemplated by the present disclosure can be specifically configured to produce any desired level of energy response 7516, in accordance with user preference and/or intended application. This can be done via a user interface communicably coupled to the control circuit 7512 (FIG. 26).

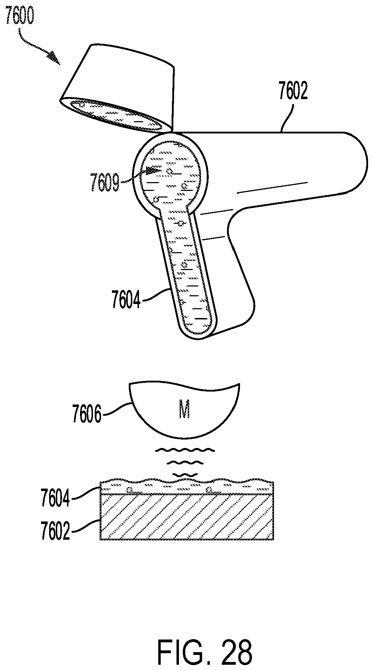

Referring now to FIG. 28, illustrating a sectioned perspective view of an adapter assembly 7602 of a surgical instrument 7600 that includes an energy management component 7604 is depicted in accordance with at least one non-limiting aspect of the present disclosure. The adapter assembly 7602 can define an internal cavity 7609 configured to accommodate a handheld device and its requisite components, such as motor 7606. According to the non-limiting aspect of FIG 28, the surgical instrument 7600 can include a material layer 7604 strategically situated throughout the internal cavity 7609, wherein the material layer 7604 is specifically configured to manage the vibrational energy generated by the motor 7606. For example, the material layer 7604 can include a butyl rubber configured to absorb vibrational energy generated by the motor 7606 when the surgical instrument 7600 is in use.

In further reference to FIG. 28, the material layer 7604 can generally include any vibration-reducing material with a sufficiently high damping coefficient and an ability to maintain performance without degradation. Accordingly, when the surgical instrument 7600 is used, the material layer 7604 can absorb shock energy and reduce the vibrations generated by the motor 7606. Additionally and/or alternatively, the material layer 7604 can include sound-deadening properties to reduce the vibrational energy impact on the surgical instrument 7600. For example, the material layer 7604 can include a material configured to absorb acoustic energy, thereby reducing the energy of sound waves generated by the motor 7606. The material layer 7604 can also be configured to absorb shock over a wide range of frequencies and temperatures. Although the non-limiting aspect of FIG. 28 includes a material layer 7604 of butyl rubber, other non-limiting aspects of the present disclosure contemplate a wide variety of material layers 7604 that possess the aforementioned properties.

Still referring to FIG. 28, the present disclosure contemplates material layers 7604 composed of any natural or synthetic materials, including visco-elastic polymers, latex, and cork, among others. Likewise, the material layer 7604 can include various mechanical components, such as springs, to assist the material layer 7604 in managing vibrational energy produced by the motor 7606. Although the non-limiting aspect of FIG. 28 includes a material layer 7604 configured to line the internal cavity 7609, still other non-limiting aspects include the material layer 7606 dispositioned within the walls of the adapter assembly 7602 itself. Accordingly, it shall be appreciated that the material layer 7604 can be intentionally dispositioned throughout the structure of the surgical instrument 7600 to accomplish a desired degree of energy management.

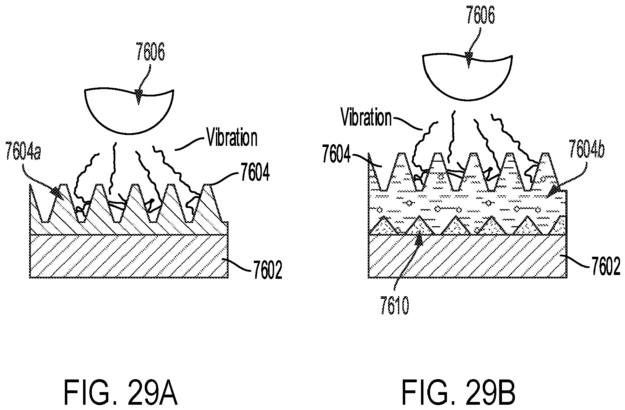

Referring now to FIG. 29A, a sectioned profile view of an alternate energy management component 7604*a* of the adapter assembly of FIG. 27 is depicted in accordance with at least one non-limiting aspect of the present disclosure. According to the non-limiting aspect of FIG 29A, the surgical instrument 7600 can include a material layer 7604a configured to manage the acoustic energy of sound waves produced by the motor 7606. For example, the material layer 7604a of FIG. 29A can include a plurality of pyramid absorbers, similar to those found in anechoic chambers. The anechoic geometry of the material layer 7604a of FIG. 29A is specifically configured to absorb and suppress the reflection of acoustic energy generated by the motor 7606 when the surgical instrument 7600 is in use. Acoustic waves emitted by the motor 7606 reflect off the angled walls of each pyramid, which prevent the energy from reflecting off the wall and back into the internal cavity 7609 (FIG. 28). In other words, the anechoic geometry prevents reverberation, which can exacerbate the vibration of the surgical instrument 7600. Accordingly, the material layer 7604a can be used to supplement and/or enhance the management of energy, thereby reducing the ensuing vibration and/or degradation of the surgical instrument 7600.

Referring now to FIG. 29B, a sectioned profile view of an alternate energy management component 7604*b* of the adapter assembly of FIG. 27 is depicted in accordance with at least one non-limiting aspect of the present disclosure. According to the non-limiting aspect of FIG 29B, the surgical instrument 7600 can include a material layer 7604a with a similar anechoic geometry depicted in FIG. 29A. The material layer 7604b manages the acoustic energy emitted by the motor 7606 similar to the material layer 7604a of FIG. 29A. However, the material layer 7604a of FIG. 29A can inadvertently insulate the adapter assembly 7602 (FIG. 28), which can be detrimental to the management of thermal energy generated by the motor 7606. Accordingly, the material layer 7604b of FIG. 29B can further include a plurality of air chambers 7610 between the material layer 7604b and an interior wall of the internal cavity 7609 of the adapter assembly 7602. As such, thermal energy can still escape the internal cavity 7609 (FIG. B28) through the plurality of air chambers 7610. Additionally and/or alternatively, the material layer 7604b to be combined with the thermally conductive channels, baffles, and heat sinks, as previously discussed. Accordingly, the material layer 7604b of FIG. 29B can be implemented to effectively manage thermal, acoustic, and vibrational energy generated by the surgical instrument 7600.

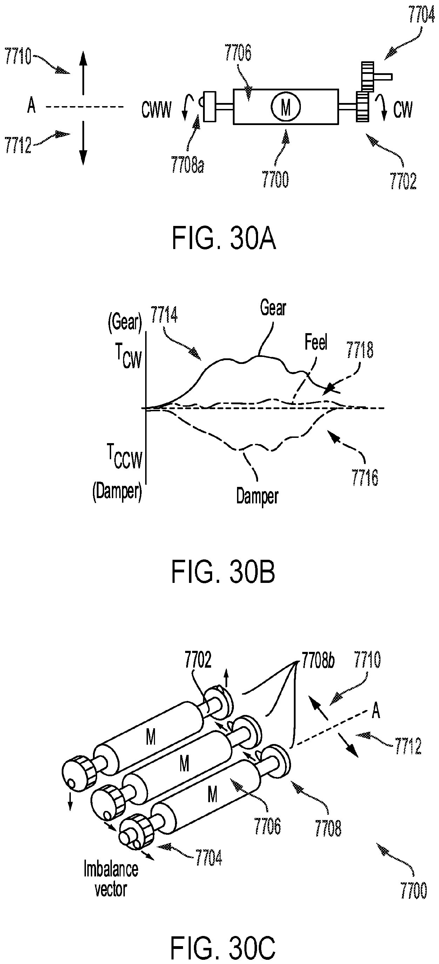

Referring now to FIG. 30A, an energy management system 7700 of a surgical instrument is depicted in accordance with at least one non-limiting aspect of the present disclosure. According to the non-limiting aspect of FIG. 30A, the energy management system 7700 can include a counterweight 7708a configured to be coupled to the driveshaft 7702 of a motor 7706 of a surgical instrument. The driveshaft 7702 of the motor 7706 traverses along a driveshaft axis A. The driveshaft axis A defines a first side 7710 and a second side 7712 of the motor 7706. In the non-limiting aspect of FIG. 30A, both the counterweight 7708a and the drive member 7704 that engages the drive shaft 7702 are both positioned on the first side 7710 of the motor 7706. Accordingly, the counterweight 7708a of the energy management system 7700 is configured to rotate in an opposite direction of the driveshaft, thereby producing a counterforce configured to dampen vibrational energy generated by the motor 7706 when the surgical instrument is in use.

Referring now to FIG. 30B, a chart depicting an energy response 7716 of the energy management system 7700 of FIG. 30A is depicted in accordance with at least one non-limiting aspect of the present disclosure. As was previously discussed, the counterforces 7716 produced by the energy management system 7700 of FIG. 30A can be specifically configured to mitigate the vibrational energy 7714 generated by the motor 7706 (FIG. 30A). The rotation of the counterweight 7708a of FIG. 30B in an opposite direction to the driveshaft 7702 can produce counterforces 7716 that are similar in magnitude-albeit opposite-to the vibrational energy 7714 generated by the motor 7706. As is depicted in FIG. 30B, the delta in magnitude between the vibrational energy 7714 generated by the motor 7706 (FIG. 30A) and the dampening energy 7716 generated by the counterweight 7708a can produce a resulting energy 7718 that can be felt by an operating clinician but is substantially lower in magnitude than the unmitigated vibrational energy 7714 generated by the motor 7706 (FIG. 30A).

Referring now to FIG. 30C, an energy management system 7700 of a surgical instrument is depicted in accordance with at least one non-limiting aspect of the present disclosure. According to the non-limiting aspect of FIG. 30C, the energy management system 7700 can include a counterweight 7708b configured to be coupled to the driveshaft 7702 of a motor 7706, similar to the non-limiting aspect of FIG. 30A. Once again, the driveshaft 7702 of the motor 7706 traverses along a driveshaft axis A, which defines a first side 7710 and a second side 7712 of the motor 7706. However, in the non-limiting aspect of FIG. 30C, the counterweight 7708b is positioned on the first side 7710 of the motor 7706 and the drive member 7704 that engages the drive shaft 7702 is positioned on the second side 7712 of the motor 7706. Accordingly, the counterweight 7708*b* of the energy management system 7700 is configured to rotate in the same direction of the driveshaft, thereby producing a counterforce configured to dampen vibrational energy generated by the motor 7706 when the surgical instrument is in use. This is exhibited in FIG. 30C via the imbalance vectors, which are oriented in an opposite direction as the force vectors produced by the counterweight 7708b dampers. Thus, the counterweight 7708*b* can produce a similar energy response to the energy response 7716 depicted in FIG. 30B, which is shown to substantially mitigate the vibrational energy 7714 (FIG. 30B) generated by the motor 7706.

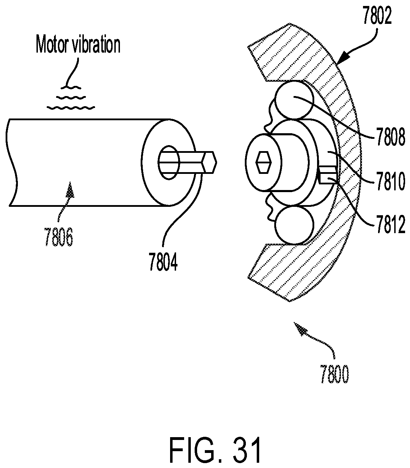

Referring now to FIG. 31, a perspective view of an energy management system 7800 of a surgical instrument is depicted in accordance with at least one non-limiting aspect of the present disclosure. According to the non-limiting aspect of FIG 31, the surgical instrument can include a motor 7806, which can include a proximal pin 7804 configured to couple to a bushing 7810 positioned within a proximal handle 7802 of the surgical instrument. The bushing 7810 can be positioned among ball bearings 7808, which enable the bushing 7810 to freely spin within the proximal handle 7802. The bushing 7810 can further include a weight 7812 configured to produce forces when the busing 7810 spins. Because the proximal pin 7804 can mechanically couple the bushing 7810 to a drive shaft of the motor 7806, the weight 7812 can be tuned to produce an energy response specifically configured to counterbalance vibrational energy generated by the motor 7806. Additionally, because the bushing 7810 can anchor the motor 7806 to the proximal handle 7802 of the surgical instrument, the motor 7806 can be inhibited from moving relative to the proximal handle 7802 of the surgical instrument. Accordingly, the bushing 7810 can produce a similar energy response to the energy response 7716 depicted in FIG. 30B, which is shown to substantially mitigate the vibrational energy 7714 (FIG. 30B) dissipated by the motor 7706.

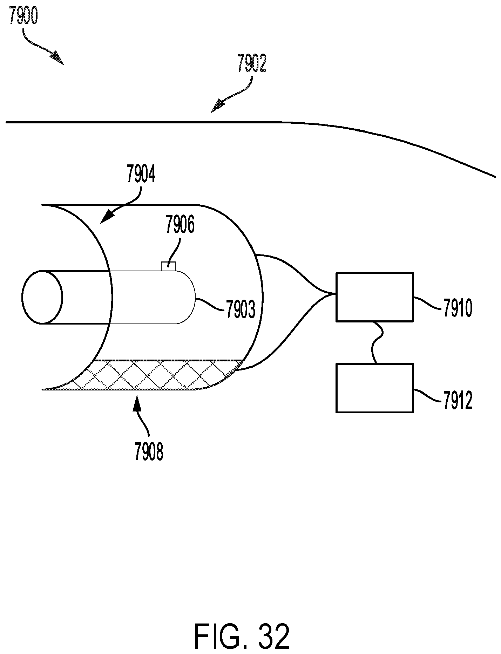

Referring now to FIG. 32, a sectioned perspective view of an energy management system 7900 of a surgical instrument is depicted in accordance with at least one non-limiting aspect of the present disclosure. According to the non-limiting aspect of FIG. 32, the energy management system 7900 can include a motor housing 7904 surrounding a motor 7903 of the handheld device 7902. According to the non-limiting aspect of FIG. 32, the motor housing 7904 can include a piezoelectric sheath 7908 that is coupled to a control circuit 7910, which is further coupled to a power source 7912. A sensor 7906 can be mechanically coupled to the motor and configured to detect the vibrational energy generated by the motor 7503 when the surgical instrument 7500 is in use.

In further reference to FIG. 32, the sensor 7906 can be further configured to generate a signal associated with the detected vibrational energy of the motor 7903. A control circuit 7910 can be coupled to the sensor 7906 and configured to receive the signal from the sensor 7906 and determine an operating level of the vibrational energy produced by the motor 7506 when the surgical instrument 7500 is in use. The control circuit 7910 can be further coupled to a power source 7912. Upon determining that the operational level of the vibrational energy produced by the motor 7903 exceeds a predetermined threshold, the control circuit 7910 can route energy from the power source 7912 to the piezoelectric sheath 7908. Upon activation, the piezoelectric sheath 7908 can be configured to generate the counterforce, thereby dampening the vibrational energy generated by the motor 7903 when the surgical instrument is in use. Accordingly, the energy management system 7900 of FIG. 32 can be configured to self-stabilize the handheld device 7902 of the surgical instrument, making it easier for an operating clinician to use.

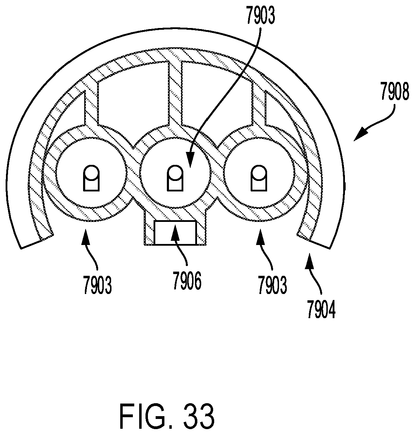

Referring now to FIG. 33, a sectioned front view of the energy management system 7900 of FIG. 32 is depicted in accordance with at least one non-limiting aspect of the present disclosure. According to the non-limiting aspect of FIG. 33, the energy management system 7900 can include a motor housing 7904 configured as a chassis that surrounds, supports, and suspends a motor 7903 assembly of the handheld device 7902 from a piezoelectric sheath 7908. As previously discussed, a sensor 7906 coupled to a control circuit 7910 (FIG. 32) and a power source 7912 (FIG. 32) is dispositioned at a predetermined location on the motor housing 7904. As can be seen in the non-limiting aspect of FIG. 33, the piezoelectric sheath 7908 can include a circumferential perimeter around the chassis specifically configured to translate a piezoelectric force uniformly throughout the chassis 7904 to mitigate-and potentially eliminate-any mechanical reactions to the vibrational energy created by the motor 7903 assembly when the surgical assembly is in use. It shall be appreciated that the chassis 7904 configuration of FIG. 33 can be attenuated depending on the number of motors and the desired reaction to the piezoelectric stimulation provided by the sheath 7908. Accordingly, any geometrical configuration can be implemented to fine-tune the performance of the energy management system 7900 in accordance with user preference and/or intended application.

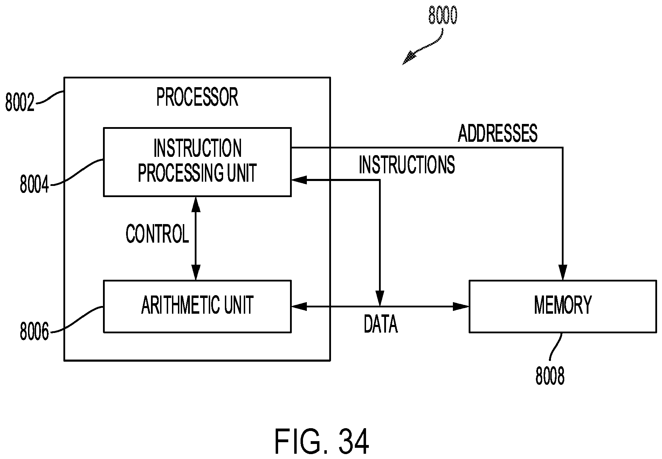

Referring now to FIG. 34, a schematic of a control circuit 8000 configured to manage energy dissipated by a surgical instrument is depicted in accordance with at least one aspect of the present disclosure. For example, the control circuit 8000 can be configured to implement the various energy management processes described herein. According to the non-limiting aspect of FIG. 34, the control circuit 8000 can include a microcontroller comprising one or more processors 8002 (e.g., microprocessor, microcontroller) coupled to at least one memory circuit 8008. The memory circuit 8008 can be configured to store machine-executable instructions that, when executed by the processor 8002, can cause the processor 8002 to execute machine instructions to implement the various processes described herein. The processor 8002 can be any one of a number of single-core or multicore processors known in the art. Alternatively and/or additionally, the microcontroller can include a logic board, such as a Field Programmable Gate Array, for example. The memory circuit 8008 can comprise volatile and non-volatile storage media. The processor 8002 may include an instruction processing unit 8004 and an arithmetic unit 8006. The instruction processing unit 8004 can be configured to receive instructions from the memory circuit 8008 of this disclosure.

The surgical instruments described herein are motivated by an electric motor; however, the surgical instrument systems described herein can be motivated in any suitable manner. In certain instances, the motors disclosed herein may comprise a portion or portions of a robotically controlled system. U.S. Patent Application Serial No. 13/118,241, entitled SURGICAL STAPLING INSTRUMENTS WITH ROTATABLE STAPLE DEPLOYMENT ARRANGEMENTS, now U.S. Patent No. 9,072,535, for example, discloses several examples of a robotic surgical instrument system in greater detail. The disclosures of International Patent Publication No. WO 2017/083125, entitled STAPLER WITH COMPOSITE CARDAN AND SCREW DRIVE, published May 18, 2017, International Patent Publication No. WO 2017/083126, entitled STAPLE PUSHER WITH LOST MOTION BETWEEN RAMPS, published May 18, 2017, International Patent Publication No. WO 2015/153642, entitled SURGICAL INSTRUMENT WITH SHIFTABLE TRANSMISSION, published October 8, 2015, U.S. Patent Application Publication No. 2017/0265954, filed March 17, 2017, entitled STAPLER WITH CABLE-DRIVEN ADVANCEABLE CLAMPING ELEMENT AND DUAL DISTAL PULLEYS, U.S. Patent Application Publication No. 2017/0265865, filed February 15, 2017, entitled STAPLER WITH CABLE-DRIVEN ADVANCEABLE CLAMPING ELEMENT AND DISTAL PULLEY, and U.S. Patent Publication No. 2017/0290586, entitled STAPLING CARTRIDGE, filed on March 29, 2017.

The surgical instruments described herein have been described in connection with the deployment and deformation of staples; however, the embodiments described herein are not so limited. Various embodiments are envisioned which deploy fasteners other than staples, such as clamps or tacks, for example. Moreover, various embodiments are envisioned which utilize any suitable means for sealing tissue. For instance, an end effector in accordance with various embodiments can comprise electrodes configured to heat and seal the tissue. Also, for instance, an end effector in accordance with certain embodiments can apply vibrational energy to seal the tissue.

Moreover, the structure of each element associated with the described forms can be alternatively described as a means for providing the function performed by the element. Also, where materials are disclosed for certain components, other materials may be used. It is therefore to be understood that the foregoing description and the appended claims are intended to cover all such modifications, combinations, and variations as falling within the scope of the disclosed forms.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and an illustrative form of the subject matter described herein applies regardless of the particular type of signal-bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer-readable media. Thus, a machine-readable medium may include any mechanism for storing or transmitting information in a form readable by a machine (e.g., a computer) but is not limited to floppy diskettes, optical disks, compact discs, read-only memory (CD-ROMs), magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical, or other forms of propagated signals (e.g., carrier waves, infrared signals, digital signals). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor comprising one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, or smart phones. Accordingly, as used herein, "control circuit" includes, but is not limited to, electrical circuitry having at least one discrete electrical circuit, electrical circuitry having at least one integrated circuit, electrical circuitry having at least one application specific integrated circuit, electrical circuitry forming a general-purpose computing device configured by a computer program (e.g., a general-purpose computer configured by a computer program that at least partially carries out processes and/or devices described herein or a microprocessor configured by a computer program that at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware, and/or circuitry configured to perform any of the aforementioned operations. Software may be embodied as a software package, code, instructions, instruction sets, and/or data recorded on non-transitory computer-readable storage medium. Firmware may be embodied as code, instructions, or instruction sets and/or data that are hard-coded (e.g., non-volatile) in memory devices.

As used in any aspect herein, the terms "component," "system," "module," and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states that may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

A network may include a packet-switched network. The communication devices may be capable of communicating with each other using a selected packet-switched network communications protocol. One example communications protocol may include an Ethernet communications protocol, which may be capable permitting communication using a Transmission Control Protocol/Internet Protocol (TCP/IP). The Ethernet protocol may comply or be compatible with the Ethernet standard published by the Institute of Electrical and Electronics Engineers (IEEE) titled "IEEE 802.3 Standard," published in December 2008 and/or later versions of this standard. Alternatively or additionally, the communication devices may be capable of communicating with each other using an X.25 communications protocol. The X.25 communications protocol may comply or be compatible with a standard promulgated by the International Telecommunication Union-Telecommunication Standardization Sector (ITU-T). Alternatively or additionally, the communication devices may be capable of communicating with each other using a frame-relay communications protocol. The frame-relay communications protocol may comply or be compatible with a standard promulgated by Consultative Committee for International Telegraph and Telephone (CCITT) and/or the American National Standards Institute (ANSI). Alternatively or additionally, the transceivers may be capable of communicating with each other using an Asynchronous Transfer Mode (ATM) communications protocol. The ATM communications protocol may comply or be compatible with an ATM standard published by the ATM Forum titled "ATM-MPLS Network Interworking 2.0" published August 2001 and/or later versions of this standard. Of course, different and/or after-developed connection-oriented network communication protocols are equally contemplated herein.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission, or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion of the surgical instrument. The term "proximal" refers to the portion closest to the clinician, and the term "distal" refers to the portion located away from the clinician. It will be further appreciated that, for convenience and clarity, spatial terms such as "vertical," "horizontal," "up," and "down" may be used herein with respect to the drawings. However, surgical instruments are used in many orientations and positions, and these terms are not intended to be limiting and/or absolute.

Those skilled in the art will recognize that, in general, terms used herein, and especially in the appended claims (e.g., bodies of the appended claims), are generally intended as "open" terms (e.g., the term "including" should be interpreted as "including, but not limited to"; the term "having" should be interpreted as "having at least"; the term "includes" should be interpreted as "includes, but is not limited to"). It will be further understood by those within the art that if a specific number of an introduced claim recitation is intended, such an intent will be explicitly recited in the claim, and in the absence of such recitation, no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations or two or more recitations). Furthermore, in those instances where a convention analogous to "at least one of A, B, and C, etc." is used, in general, such a construction is intended in the sense one having skill in the art would understand the convention (e.g., "a system having at least one of A, B, and C" would include, but not be limited to, systems that have A alone, B alone, C alone, A and B together, A and C together, B and C together, and/or A, B, and C together). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A," "B," or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures, or characteristics may be combined in any suitable manner in one or more aspects.

In summary, numerous benefits have been described that result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

## Claims

1. An adapter assembly (6001, 6100, 6302, 6500, 7502) configured to, at least partially, encase a handheld device (6016, 6304, 6504) of a surgical instrument (6000) configured for use with a plurality of interchangeable end effectors (6008), wherein the handheld device comprises a power source and a drive assembly, the adapter assembly comprising:
an outer housing comprising an internal cavity (6209, 6109) configured to encase the handheld device;
a drive interface assembly comprising an internal interface configured to mechanically engage the drive assembly of the handheld device, and an external interface configured to mechanically engage a drive assembly of an interchangeable end effector; and **characterised by**
an energy management system (7504, 7700) configured to manage energy dissipated by the handheld device when the surgical instrument is in use.

2. The adapter assembly (6100, 6500) of claim 1, wherein the outer housing further comprises a proximal portion (6102, 6502) and a distal portion (6104, 6503) rotatably coupled to the proximal portion via a hinge (6106), wherein the distal portion is configured to move relative to the proximal portion between an open configuration and a closed configuration, wherein a sterile barrier is established around the handheld device (6504) in the closed configuration, and wherein the energy management system comprises:
a first heat sink (6114, 6510) coupled to the proximal portion of the outer housing, wherein the first heat sink is configured for mechanical contact with the handheld device when the handheld device is encased within the internal cavity of the adapter assembly; and
a second heat sink (6108, 6508) coupled to an exterior surface of the distal portion of the outer housing, wherein the second heat sink is configured to mechanically contact the first heat sink without disrupting the sterile barrier when the adapter assembly is in the closed configuration, and wherein mechanical contact between the first heat sink and the second heat sink creates a thermally conductive path between the handheld device and the second heat sink.

3. The adapter assembly (6302) of claim 1, wherein the energy management system further comprises:
a piezoelectric fan (6310) coupled to the power source (6311);
a temperature sensor (6308) configured to generate signals associated with an operating temperature of the handheld device (6304); and
a control circuit (6309) coupled to the power source and the energy management system, wherein the control circuit is configured to:
receive a first signal from the temperature sensor;
determine a first operating temperature of the handheld device based, at least in part, on the first signal;
determine if the first operating temperature meets or exceeds a predetermined threshold; and
cause the piezoelectric fan to oscillate upon determining that the first operating temperature meets or exceeds the predetermined threshold.

4. The adapter assembly (7502) of claim 1, wherein the energy management system (7504) comprises:
a sensor (7510) configured to detect vibrations and generate a signal associated with a vibration of the surgical instrument;
a piezoelectric dampener (7504) configured to generate dampening vibrations; and
a control circuit (7512) coupled to the power source and the energy management system, wherein the control circuit is configured to:
receive a first signal from the sensor;
determine an operating vibration level of the surgical instrument based, at least in part, on the signal received from the sensor;
determine if the operating vibration level of the surgical instrument exceeds a predetermined threshold; and
cause the piezoelectric dampener to produce a dampening vibration based, at least in part, on the determination that the operating vibration level of the surgical instrument exceeds the predetermined threshold.

5. The adapter assembly of claim 1, wherein the first drive assembly comprises a motor (7706) and a drive member (7704), wherein the motor comprises a drive shaft (7702) and and the drive member is configured to engage the drive shaft, and
wherein the energy management system (7700) comprises a dampening component (7708a), wherein the drive member and the dampening component are coupled to a drive shaft of the motor, wherein the drive shaft defines a first side (7710) and a second side (7712) of the motor, wherein the dampening component and the drive member are both positioned on the first side of the motor and configured to rotate in opposite directions, wherein the rotation of the drive emember applies a first force on the motor and the rotation of the dampening component applies a second force on the motor in a direction opposite to that of the first force, thereby reducing a net energy dissipated by the handheld device.

6. A surgical instrument (6000, 6300, 7600), comprising:
an adapter assembly (6001, 6100, 6302, 6500, 7502, 7602) according to any one of claims 1 to 5;
the handheld device (6016, 6304) comprising the power source and the first drive assembly, wherein the first drive assembly comprises a first operating mode; and
an interchangeable end effector (6008) comprising a second drive assembly, wherein the second drive assembly comprises a second operating mode that is different than the first operating mode of the first drive assembly, and wherein the second drive assembly is configured to mechanically couple to the external interface of the drive interface;
wherein the internal interface of the drive interface assembly is configured to transfer a motion generated by the first drive assembly to the external interface of the drive interface assembly, and wherein the external interface of the drive interface assembly is configured to transfer a motion of the inner interface of the drive interface assembly to the second drive assembly of the interchangeable end effector.

7. The surgical instrument (6000) of claim 6, wherein the surgical instrument comprises the adapter assembly (6500) according to claim 2, and wherein the surgical instrument further comprises a thermal paste (6516) positioned between the first heat sink (6510) and the second heat sink (6508), wherein the thermal paste is configured to increase a surface area of the interface between the first heat sink and the second heat sink and, therefore, enhance a conductive efficiency of the interface.

8. The surgical instrument (6300) of claim 6, wherein the surgical instrument comprises the adapter assembly (6302) according to claim 3, and wherein the control circuit (6309) is further configured to:
receive a second signal from the temperature sensor (6308);
determine a second operating temperature of the handheld device (6304) based, at least in part, on the second signal;
compare the first operating temperature to the second operating temperature; and
vary the oscillation of the piezoelectric fan (6310) based, at least in part, on the comparison of the first operating temperature and the second operating temperature.

9. The surgical instrument of claim 6, wherein the surgical instrument comprises the adapter assembly according to claim 1, and wherein the first drive assembly comprises a motor (7706), a drive member (7704), wherein the motor comprises a drive shaft (7702) and the drive member is configured to engage the drive shaft, and
wherein the energy management system (7770) comprises a dampening component (7708b), wherein the drive member and the dampening component are coupled to the drive shaft of the motor, wherein the drive shaft defines a first side (7710) and a second side (7712) of the motor, wherein the dampening component is positioned on the first side of the motor and the drive member is positioned on the second side of the motor, wherein the drive member and the dampening component are configured to rotate in the same direction such that the rotation of the drive member applies a first force on the motor and the rotation of the dampening component applies a second force on the motor in a direction opposite to that of the first force, thereby reducing a net energy dissipated by the handheld device.

10. The surgical instrument (7600) of claim 6, wherein the surgical instrument comprises the adapter assembly (7602) according to claim 1, and wherein the energy management system comprises a material (7604) dispositioned on, at least a portion of, a wall of the internal cavity (7609), wherein the material is configured to dampen acoustic vibrations generated by the first drive assembly, optionally wherein the material comprises at least one of a butyl rubber, an asphalt, and an acoustic energy dampening spray, or any combination thereof.

11. The surgical instrument (7600) of claim 6, wherein the surgical instrument comprises the adapter assembly (7602) according to claim 1, and wherein the energy management system comprises a geometric feature (7604a, 7604b) dispositioned on, at least a portion of, a wall of the internal cavity (7609), wherein the geometric feature is configured to dampen acoustic energy generated by the first drive assembly.

12. The surgical instrument (7600) of claim 11, wherein the geometric feature (7604b) comprises a plurality of anechoic chambers, wherein each anechoic chamber comprises a plurality of air pockets (7610), and wherein the geometric feature is dispositioned such that air can flow between the wall of the internal cavity and, at least a portion of, each anechoic chamber of the plurality of anechoic chambers.

13. A surgical instrument (6000, 6700), comprising:
a handheld device (6016, 6702) comprising a first drive assembly and a power source;
an adapter assembly (6001) comprising an internal cavity configured to accommodate a handheld device, wherein the adapter assembly is configured to establish a sterile barrier around the handheld device; and **characterised by**
an energy management system configured to extract energy dissipated by the handheld device from the internal cavity without disrupting the sterile barrier.

14. The surgical instrument (6000, 6700) of claim 13, further comprising:
a plurality of interchangeable end effectors (6008), wherein a first interchangeable end effector of the plurality of interchangeable end effectors comprises a first drive interface configured for a first operating mode, and wherein a second interchangeable end effector of the plurality of interchangeable end effectors comprises a second drive interface configured for a second operating mode, wherein the first operating mode is different than the second operating mode; and
a drive interface assembly comprising an internal interface configured to engage the first drive assembly and an external interface configured to engage the first drive interface and the second drive interface.

15. The surgical instrument (6700) of claim 14, wherein the adapter assembly further comprises a proximal portion and a distal portion rotatably coupled to the proximal portion via a hinge, wherein the distal portion (6704) is configured to move relative to the proximal portion between an open configuration and a closed configuration, and wherein the energy management system comprises:
a first heat sink (6708) coupled to the proximal portion, wherein the first heat sink is configured for mechanical contact with the handheld device (6702); and
a second heat sink (6712) coupled to an exterior surface of the distal portion (6704) of the outer housing, wherein the second heat sink is configured to mechanically contact the first heat sink without disrupting the sterile barrier when the adapter assembly is in the closed configuration, and wherein mechanical contact between the first heat sink and the second heat sink creates a thermally conductive path between the handheld device and the second heat sink.

## Patentansprüche

1. Adapterbaugruppe (6001, 6100, 6302, 6500, 7502), die dazu ausgestaltet ist, eine in der Hand gehaltene Vorrichtung (6016, 6304, 6504) eines chirurgischen Instruments (6000), die zur Verwendung mit einer Vielzahl von austauschbaren Endeffektoren (6008) ausgestaltet ist, mindestens teilweise zu umschließen, wobei die in der Hand gehaltene Vorrichtung eine Energiequelle und eine Antriebsbaugruppe umfasst, wobei die Adapterbaugruppe Folgendes umfasst:
ein äußeres Gehäuse, das einen inneren Hohlraum (6209, 6109) umfasst, der zum Umschließen der in der Hand gehaltenen Vorrichtung ausgestaltet ist,
eine Antriebsschnittstellenbaugruppe, die eine innere Schnittstelle, die zur mechanischen Ineingriffnahme der Antriebsbaugruppe der in der Hand gehaltenen Vorrichtung ausgestaltet ist, und eine äußere Schnittstelle, die zur mechanischen Ineingriffnahme einer Antriebsbaugruppe eines austauschbaren Endeffektors ausgestaltet ist, umfasst, **gekennzeichnet durch**
ein Energieverwaltungssystem (7504, 7700), das dazu ausgestaltet ist, Energie, die von der in der Hand gehaltenen Vorrichtung abgegeben wird, wenn das chirurgische Instrument im Gebrauch ist, zu verwalten.

2. Adapterbaugruppe (6100, 6500) nach Anspruch 1, wobei das äußere Gehäuse ferner einen proximalen Abschnitt (6102, 6502) und einen über ein Scharnier (6106) drehbar an den proximalen Abschnitt gekoppelten distalen Abschnitt (6104, 6503) umfasst, wobei der distale Abschnitt dazu ausgestaltet ist, sich bezüglich des proximalen Abschnitts zwischen einer offenen Konfiguration und einer geschlossenen Konfiguration zu bewegen, wobei eine Sterilbarriere in der geschlossenen Konfiguration um die in der Hand gehaltene Vorrichtung (6504) herum errichtet ist und wobei das Energieverwaltungssystem Folgendes umfasst:
einen ersten Kühlkörper (6114, 6510), der an den proximalen Abschnitt des äußeren Gehäuses gekoppelt ist, wobei der erste Kühlkörper für mechanischen Kontakt mit der in der Hand gehaltenen Vorrichtung ausgestaltet ist, wenn die in der Hand gehaltene Vorrichtung in dem inneren Hohlraum der Adapterbaugruppe umschlossen ist, und
einen zweiten Kühlkörper (6108, 6508), der an eine Außenfläche des distalen Abschnitts des äußeren Gehäuses gekoppelt ist, wobei der zweite Kühlkörper dazu ausgestaltet ist, den ersten Kühlkörper mechanisch ohne Störung der Sterilbarriere zu kontaktieren, wenn die Adapterbaugruppe in der geschlossenen Konfiguration ist, und wobei mechanischer Kontakt zwischen dem ersten Kühlkörper und dem zweiten Kühlkörper einen Wärmeleitpfad zwischen der in der Hand gehaltenen Vorrichtung und dem zweiten Kühlkörper erzeugt.

3. Adapterbaugruppe (6302) nach Anspruch 1, wobei das Energieverwaltungssystem ferner Folgendes umfasst:
einen an die Energiequelle (6311) gekoppelten Piezo-Ventilator (6310),
einen Temperatursensor (6308), der dazu ausgestaltet ist, einer Betriebstemperatur der in der Hand gehaltenen Vorrichtung (6304) zugeordnete Signale zu erzeugen, und
eine Steuerschaltung (6309), die an die Energiequelle und das Energieverwaltungssystem gekoppelt ist, wobei die Steuerschaltung dazu ausgestaltet ist,
ein erstes Signal von dem Temperatursensor zu empfangen,
mindestens teilweise auf der Grundlage des ersten Signals eine erste Betriebstemperatur der in der Hand gehaltenen Vorrichtung zu bestimmen,
zu bestimmen, ob die erste Betriebstemperatur einen vorbestimmten Schwellenwert erreicht oder überschreitet, und
bei Bestimmung, dass die erste Betriebstemperatur den vorbestimmten Schwellenwert erreicht oder überschreitet, das Oszillieren des Piezo-Ventilators zu veranlassen.

4. Adapterbaugruppe (7502) nach Anspruch 1, wobei das Energieverwaltungssystem (7504) Folgendes umfasst:
einen Sensor (7510), der dazu ausgestaltet ist, Vibrationen zu erfassen und ein einer Vibration des chirurgischen Instruments zugeordnetes Signal zu erzeugen,
eine piezoelektrische Dämpfungsvorrichtung (7504), die zur Erzeugung von Dämpfungsschwingungen ausgestaltet ist, und
eine Steuerschaltung (7512), die an die Energiequelle und das Energieverwaltungssystem gekoppelt ist,
wobei die Steuerschaltung dazu ausgestaltet ist,
ein erstes Signal von dem Sensor zu empfangen,
mindestens teilweise auf der Grundlage des von dem Sensor empfangenen Signals ein Betriebsvibrationsniveau des chirurgischen Instruments zu bestimmen,
zu bestimmen, ob das Betriebsvibrationsniveau des chirurgischen Instruments einen vorbestimmten Schwellenwert überschreitet, und
mindestens teilweise auf der Grundlage der Bestimmung, dass das Betriebsvibrationsniveau des chirurgischen Instruments den vorbestimmten Schwellenwert überschreitet, zu veranlassen, dass die piezoelektrische Dämpfungsvorrichtung eine Dämpfungsschwingung erzeugt.

5. Adapterbaugruppe nach Anspruch 1, wobei die erste Antriebsbaugruppe einen Motor (7706) und ein Antriebsglied (7704) umfasst, wobei der Motor eine Antriebswelle (7702) umfasst und das Antriebsglied zur Ineingriffnahme der Antriebswelle ausgestaltet ist, und
wobei das Energieverwaltungssystem (7700) eine Dämpfungskomponente (7708a) umfasst, wobei das Antriebsglied und die Dämpfungskomponente an eine Antriebswelle des Motors gekoppelt sind, wobei die Antriebswelle eine erste Seite (7710) und eine zweite Seite (7712) des Motors definiert, wobei sowohl die Dämpfungskomponente als auch das Antriebsglied an der ersten Seite des Motors positioniert und dazu ausgestaltet sind, sich in entgegengesetzten Richtungen zu drehen, wobei der Motor durch die Drehung des Antriebsglieds mit einer ersten Kraft beaufschlagt wird und der Motor durch die Drehung der Dämpfungskomponente in einer zu der ersten Kraft entgegengesetzten Richtung mit einer zweiten Kraft beaufschlagt wird, wodurch eine von der in der Hand gehaltenen Vorrichtung abgegebene Nettoenergie reduziert wird.

6. Chirurgisches Instrument (6000, 6300, 7600), umfassend:
eine Adapterbaugruppe (6001, 6100, 6302, 6500, 7502) nach einem der Ansprüche 1 bis 5,
die in der Hand gehaltene Vorrichtung (6016, 6304), die die Energiequelle und die erste Antriebsbaugruppe umfasst, wobei die erste Antriebsbaugruppe einen ersten Betriebsmodus umfasst, und
einen austauschbaren Endeffektor (6008), der eine zweite Antriebsbaugruppe umfasst, wobe die zweite Antriebsbaugruppe einen zweiten Betriebsmodus umfasst, der von dem ersten Betriebsmodus der ersten Antriebsbaugruppe verschieden ist, und wobei die zweite Antriebsbaugruppe zur mechanischen Koppelung an die äußere Schnittstelle der Antriebsschnittstelle ausgestaltet ist,
wobei die innere Schnittstelle der Antriebsschnittstellenbaugruppe dazu ausgestaltet ist, eine von der ersten Antriebsbaugruppe erzeugte Bewegung an die äußere Schnittstelle der Antriebsschnittstellenbaugruppe zu übertragen, und wobei die äußere Schnittstelle der Antriebsschnittstellenbaugruppe dazu ausgestaltet ist, eine Bewegung der inneren Schnittstelle der Antriebsschnittstellenbaugruppe an die zweite Antriebsbaugruppe des austauschbaren Endeffektors zu übertragen.

7. Chirurgisches Instrument (6000) nach Anspruch 6, wobei das chirurgische Instrument die Adapterbaugruppe (6500) nach Anspruch 2 umfasst und wobei das chirurgische Instrument ferner eine zwischen dem ersten Kühlkörper (6510) und dem zweiten Kühlkörper (6508) positionierte Wärmeleitpaste (6516) umfasst, wobei die Wärmeleitpaste dazu ausgestaltet ist, eine Fläche der Schnittstelle zwischen dem ersten Kühlkörper und dem zweiten Kühlkörper zu vergrößern und dadurch eine Leitfähigkeitseffizienz der Schnittstelle zu verbessern.

8. Chirurgisches Instrument (6300) nach Anspruch 6, wobei das chirurgische Instrument die Adapterbaugruppe (6302) nach Anspruch 3 umfasst und wobei die Steuerschaltung (6309) ferner dazu ausgestaltet ist,
ein zweites Signal von dem Temperatursensor (6308) zu empfangen,
mindestens teilweise auf der Grundlage des zweiten Signals eine zweite Betriebstemperatur der in der Hand gehaltenen Vorrichtung (6304) zu bestimmen,
die erste Betriebstemperatur mit der zweiten Betriebstemperatur zu vergleichen und
mindestens teilweise auf der Grundlage des Vergleichs der ersten Betriebstemperatur mit der zweiten Betriebstemperatur die Oszillation des Piezo-Ventilators (6310) zu variieren.

9. Chirurgisches Instrument nach Anspruch 6, wobei das chirurgische Instrument die Adapterbaugruppe nach Anspruch 1 umfasst und wobei die erste Antriebsbaugruppe einen Motor (7706) und ein Antriebsglied (7704) umfasst, wobei der Motor eine Antriebswelle (7702) umfasst und das Antriebsglied zur Ineingriffnahme der Antriebswelle ausgestaltet ist, und
wobei das Energieverwaltungssystem (7770) eine Dämpfungskomponente (7708a) umfasst, wobei das Antriebsglied und die Dämpfungskomponente an eine Antriebswelle des Motors gekoppelt sind, wobei die Antriebswelle eine erste Seite (7710) und eine zweite Seite (7712) des Motors definiert, wobei die Dämpfungskomponente an der ersten Seite des Motors positioniert ist und das Antriebsglied an der zweiten Seite des Motors positioniert ist, wobei das Antriebsglied und die Dämpfungskomponente dazu ausgestaltet sind, sich in der gleichen Richtung zu drehen, so dass der Motor durch die Drehung des Antriebsglieds mit einer ersten Kraft beaufschlagt wird und der Motor durch die Drehung der Dämpfungskomponente in einer zu der ersten Kraft entgegengesetzten Richtung mit einer zweiten Kraft beaufschlagt wird, wodurch eine von der in der Hand gehaltenen Vorrichtung abgegebene Nettoenergie reduziert wird.

10. Chirurgisches Instrument (7600) nach Anspruch 6, wobei das chirurgische Instrument die Adapterbaugruppe (7602) nach Anspruch 1 umfasst und wobei das Energieverwaltungssystem ein Material (7604) umfasst, das auf mindestens einem Abschnitt einer Wand des inneren Hohlraums (7609) positioniert ist, wobei das Material zur Dämpfung von durch die erste Antriebsbaugruppe erzeugten akustischen Schwingungen ausgestaltet ist, optional wobei das Material einen Butylkautschuk und/oder einen Asphalt und/oder ein Spray zum Dämpfen von akustischer Energie und/oder eine beliebige Kombination davon umfasst.

11. Chirurgisches Instrument (7600) nach Anspruch 6, wobei das chirurgische Instrument die Adapterbaugruppe (7602) nach Anspruch 1 umfasst und wobei das Energieverwaltungssystem ein geometrisches Merkmal (7604a, 7604b) umfasst, das auf mindestens einem Abschnitt einer Wand des inneren Hohlraums (7609) positioniert ist, wobei das geometrische Merkmal zur Dämpfung der von der ersten Antriebsbaugruppe erzeugten akustischen Energie ausgestaltet ist.

12. Chirurgisches Instrument (7600) nach Anspruch 11, wobei das geometrische Merkmal (7604b) eine Vielzahl von schalltoten Kammern umfasst, wobei jede schalltote Kammer eine Vielzahl von Luftkammern (7610) umfasst und wobei das geometrische Merkmal so positioniert ist, dass Luft zwischen der Wand des inneren Hohlraums und mindestens einem Abschnitt jeder schalltoten Kammer der Vielzahl von schalltoten Kammern strömen kann.

13. Chirurgisches Instrument (6000, 6700), umfassend:
eine in der Hand gehaltene Vorrichtung (6016, 6702), die eine erste Antriebsbaugruppe und eine Energiequelle umfasst,
eine Adapterbaugruppe (6001), die einen inneren Hohlraum umfasst, der zur Aufnahme einer in der Hand gehaltenen Vorrichtung ausgestaltet ist, wobei die Adapterbaugruppe zur Errichtung einer Sterilbarriere um die in der Hand gehaltene Vorrichtung herum ausgestaltet ist, **gekennzeichnet durch**
ein Energieverwaltungssystem, das dazu ausgestaltet ist, von der in der Hand gehaltenen Vorrichtung abgegebene Energie aus dem inneren Hohlraum ohne Störung der Sterilbarriere abzuziehen.

14. Chirurgisches Instrument (6000, 6700) nach Anspruch 13, ferner umfassend:
eine Vielzahl von austauschbaren Endeffektoren (6008), wobei ein erster austauschbarer Endeffektor der Vielzahl von austauschbaren Endeffektoren eine für einen ersten Betriebsmodus ausgestaltete erste Antriebsschnittstelle umfasst und wobei ein zweiter austauschbarer Endeffektor der Vielzahl von austauschbaren Endeffektoren eine für einen zweiten Betriebsmodus ausgestaltete zweite Antriebsschnittstelle umfasst, wobei der erste Betriebsmodus von dem zweiten Betriebsmodus verschieden ist, und
eine Antriebsschnittstellenbaugruppe, die eine zur Ineingriffnahme der ersten Antriebsbaugruppe ausgestaltete innere Schnittstelle und eine zur Ineingriffnahme der ersten Antriebsschnittstelle und der zweiten Antriebsschnittstelle ausgestaltete äußere Schnittstelle umfasst.

15. Chirurgisches Instrument (6700) nach Anspruch 14, wobei die Adapterbaugruppe ferner einen proximalen Abschnitt und einen über ein Scharnier drehbar an den proximalen Abschnitt gekoppelten distalen Abschnitt umfasst, wobei der distale Abschnitt (6704) dazu ausgestaltet ist, sich bezüglich des proximalen Abschnitts zwischen einer offenen Konfiguration und einer geschlossenen Konfiguration zu bewegen, und wobei das Energieverwaltungssystem Folgendes umfasst:
einen ersten Kühlkörper (6708), der an den proximalen Abschnitt gekoppelt ist, wobei der erste Kühlkörper für mechanischen Kontakt mit der in der Hand gehaltenen Vorrichtung (6702) ausgestaltet ist, und
einen zweiten Kühlkörper (6712), der an eine Außenfläche des distalen Abschnitts (6704) des äußeren Gehäuses gekoppelt ist, wobei der zweite Kühlkörper dazu ausgestaltet ist, den ersten Kühlkörper mechanisch ohne Störung der Sterilbarriere zu kontaktieren, wenn die Adapterbaugruppe in der geschlossenen Konfiguration ist, und wobei mechanischer Kontakt zwischen dem ersten Kühlkörper und dem zweiten Kühlkörper einen Wärmeleitpfad zwischen der in der Hand gehaltenen Vorrichtung und dem zweiten Kühlkörper erzeugt.

## Revendications

1. Ensemble adaptateur (6001, 6100, 6302, 6500, 7502) configuré pour, au moins partiellement, envelopper un dispositif portatif (6016, 6304, 6504) d'un instrument chirurgical (6000) configuré pour être utilisé avec une pluralité d'effecteurs terminaux interchangeables (6008), le dispositif portatif comprenant une source d'alimentation et un ensemble d'entraînement, l'ensemble adaptateur comprenant :
un boîtier externe comprenant une cavité interne (6209, 6109) configurée pour envelopper le dispositif portatif ;
un ensemble d'interface d'entraînement comprenant une interface interne configurée pour venir en prise mécaniquement avec l'ensemble d'entraînement du dispositif portatif, et une interface externe configurée pour venir en prise mécaniquement avec un ensemble d'entraînement d'un effecteur terminal interchangeable ; et **caractérisé par**
un système de gestion de l'énergie (7504, 7700) configuré pour gérer l'énergie dissipée par le dispositif portatif lorsque l'instrument chirurgical est en cours d'utilisation.

2. Ensemble adaptateur (6100, 6500) selon la revendication 1, le boîtier externe comprenant en outre une partie proximale (6102, 6502) et une partie distale (6104, 6503) couplée de manière rotative à la partie proximale par l'intermédiaire d'une charnière (6106), la partie distale étant configurée pour se déplacer par rapport à la partie proximale entre une configuration ouverte et une configuration fermée, une barrière stérile étant établie autour du dispositif portatif (6504) dans la configuration fermée, et le système de gestion de l'énergie comprenant :
un premier dissipateur thermique (6114, 6510) couplé à la partie proximale du boîtier externe, le premier dissipateur thermique étant configuré pour un contact mécanique avec le dispositif portatif lorsque le dispositif portatif est enveloppé dans la cavité interne de l'ensemble adaptateur ; et
un second dissipateur thermique (6108, 6508) couplé à une surface extérieure de la partie distale du boîtier externe, le second dissipateur thermique étant configuré pour entrer en contact mécanique avec le premier dissipateur thermique sans rompre la barrière stérile lorsque l'ensemble adaptateur est dans la configuration fermée, et le contact mécanique entre le premier dissipateur thermique et le second dissipateur thermique créant un chemin thermiquement conducteur entre le dispositif portatif et le second dissipateur thermique.

3. Ensemble adaptateur (6302) selon la revendication 1, le système de gestion de l'énergie comprenant en outre :
un ventilateur piézoélectrique (6310) couplé à la source d'alimentation (6311) ;
un capteur de température (6308) configuré pour générer des signaux associés à une température de fonctionnement du dispositif portatif (6304) ; et
un circuit de commande (6309) couplé à la source d'alimentation et au système de gestion de l'énergie, le circuit de commande étant configuré pour :
recevoir un premier signal du capteur de température ;
déterminer une première température de fonctionnement du dispositif portatif sur la base, au moins en partie, du premier signal ;
déterminer si la première température de fonctionnement atteint ou dépasse un seuil prédéterminé ; et
amener le ventilateur piézoélectrique à osciller après avoir déterminé que la première température de fonctionnement atteint ou dépasse le seuil prédéterminé.

4. Ensemble adaptateur (7502) selon la revendication 1, le système de gestion de l'énergie (7504) comprenant :
un capteur (7510) configuré pour détecter des vibrations et générer un signal associé à une vibration de l'instrument chirurgical ;
un amortisseur piézoélectrique (7504) configuré pour générer des vibrations d'amortissement ; et
un circuit de commande (7512) couplé à la source d'alimentation et au système de gestion de l'énergie, le circuit de commande étant configuré pour :
recevoir un premier signal du capteur ;
déterminer un niveau de vibration de fonctionnement de l'instrument chirurgical sur la base, au moins en partie, du signal reçu du capteur ;
déterminer si le niveau de vibration de fonctionnement de l'instrument chirurgical dépasse un seuil prédéterminé ; et
amener l'amortisseur piézoélectrique à produire une vibration d'amortissement sur la base, au moins en partie, de la détermination que le niveau de vibration de fonctionnement de l'instrument chirurgical dépasse le seuil prédéterminé.

5. Ensemble adaptateur selon la revendication 1, le premier ensemble d'entraînement comprenant un moteur (7706) et un élément d'entraînement (7704), le moteur comprenant un arbre d'entraînement (7702) et l'élément d'entraînement étant configuré pour venir en prise avec l'arbre d'entraînement, et
le système de gestion de l'énergie (7700) comprenant un composant d'amortissement (7708a), l'élément d'entraînement et le composant d'amortissement étant couplés à un arbre d'entraînement du moteur, l'arbre d'entraînement définissant un premier côté (7710) et un second côté (7712) du moteur, le composant d'amortissement et l'élément d'entraînement étant tous deux positionnés sur le premier côté du moteur et configurés pour tourner dans des directions opposées, la rotation de l'élément d'entraînement appliquant une première force sur le moteur et la rotation du composant d'amortissement appliquant une seconde force sur le moteur dans une direction opposée à celle de la première force, réduisant ainsi une énergie nette dissipée par le dispositif portatif.

6. Instrument chirurgical (6000, 6300, 7600) comprenant :
un ensemble adaptateur (6001, 6100, 6302, 6500, 7502, 7602) selon l'une quelconque des revendications 1 à 5 ;
le dispositif portatif (6016, 6304) comprenant la source d'alimentation et le premier ensemble d'entraînement, le premier ensemble d'entraînement comprenant un premier mode de fonctionnement, et
un effecteur terminal interchangeable (6008) comprenant un second ensemble d'entraînement, le second ensemble d'entraînement comprenant un second mode de fonctionnement qui est différent du premier mode de fonctionnement du premier ensemble d'entraînement, et le second ensemble d'entraînement étant configuré pour se coupler mécaniquement à l'interface externe de l'interface d'entraînement ;
l'interface interne de l'ensemble d'interface d'entraînement étant configurée pour transférer un mouvement généré par le premier ensemble d'entraînement à l'interface externe de l'ensemble d'interface d'entraînement, et l'interface externe de l'ensemble d'interface d'entraînement étant configurée pour transférer un mouvement de l'interface interne de l'ensemble d'interface d'entraînement au second ensemble d'entraînement de l'effecteur terminal interchangeable.

7. Instrument chirurgical (6000) selon la revendication 6, l'instrument chirurgical comprenant l'ensemble adaptateur (6500) selon la revendication 2, et l'instrument chirurgical comprenant en outre une pâte thermique (6516) positionnée entre le premier dissipateur thermique (6510) et le second dissipateur thermique (6508), la pâte thermique étant configurée pour augmenter une surface de l'interface entre le premier dissipateur thermique et le second dissipateur thermique et, par conséquent, améliorer l'efficacité conductrice de l'interface.

8. Instrument chirurgical (6300) selon la revendication 6, l'instrument chirurgical comprenant l'ensemble adaptateur (6302) selon la revendication 3, et le circuit de commande (6309) étant en outre configuré pour :
recevoir un second signal du capteur de température (6308) ;
déterminer une seconde température de fonctionnement du dispositif portatif (6304) sur la base, au moins en partie, du second signal ;
comparer la première température de fonctionnement à la seconde température de fonctionnement ; et
faire varier l'oscillation du ventilateur piézoélectrique (6310) sur la base, au moins en partie, de la comparaison entre la première température de fonctionnement et la seconde température de fonctionnement.

9. Instrument chirurgical selon la revendication 6, l'instrument chirurgical comprenant l'ensemble adaptateur selon la revendication 1, et le premier ensemble d'entraînement comprenant un moteur (7706), un élément d'entraînement (7704), le moteur comprenant un arbre d'entraînement (7702) et l'élément d'entraînement étant configuré pour venir en prise avec l'arbre d'entraînement, et
le système de gestion de l'énergie (7770) comprenant un composant d'amortissement (7708b), l'élément d'entraînement et le composant d'amortissement étant couplés à l'arbre d'entraînement du moteur, l'arbre d'entraînement définissant un premier côté (7710) et un second côté (7712) du moteur, le composant d'amortissement étant positionné sur le premier côté du moteur et l'élément d'entraînement étant positionné sur le second côté du moteur, l'élément d'entraînement et le composant d'amortissement étant configurés pour tourner dans le même sens de sorte que la rotation de l'élément d'entraînement applique une première force sur le moteur et la rotation du composant d'amortissement applique une seconde force sur le moteur dans une direction opposée à celle de la première force, réduisant ainsi une énergie nette dissipée par le dispositif portatif.

10. Instrument chirurgical (7600) selon la revendication 6, l'instrument chirurgical comprenant l'ensemble adaptateur (7602) selon la revendication 1, et le système de gestion de l'énergie comprenant un matériau (7604) disposé sur, au moins une partie d'une paroi de la cavité interne (7609), le matériau étant configuré pour amortir des vibrations acoustiques générées par le premier ensemble d'entraînement, éventuellement le matériau comprenant au moins l'un parmi un caoutchouc butyle, un asphalte, et une pulvérisation d'amortissement de l'énergie acoustique, ou toute combinaison de ceux-ci.

11. Instrument chirurgical (7600) selon la revendication 6, l'instrument chirurgical comprenant l'ensemble adaptateur (7602) selon la revendication 1, et le système de gestion de l'énergie comprenant une caractéristique géométrique (7604a, 7604b) disposée sur, au moins une partie de, une paroi de la cavité interne (7609), la caractéristique géométrique étant configurée pour amortir l'énergie acoustique générée par le premier ensemble d'entraînement.

12. Instrument chirurgical (7600) selon la revendication 11, la caractéristique géométrique (7604b) comprenant une pluralité de chambres anéchoïques, chaque chambre anéchoïque comprenant une pluralité de poches d'air (7610), et la caractéristique géométrique étant disposée de manière à ce que l'air puisse circuler entre la paroi de la cavité interne et, au moins une partie, de chaque chambre anéchoïque de la pluralité de chambres anéchoïques.

13. Instrument chirurgical (6000, 6700) comprenant :
un dispositif portatif (6016, 6702) comprenant un premier ensemble d'entraînement et une source d'alimentation ;
un ensemble adaptateur (6001) comprenant une cavité interne configurée pour accueillir un dispositif portatif, l'ensemble adaptateur étant configuré pour établir une barrière stérile autour du dispositif portatif ; et **caractérisé par**
un système de gestion de l'énergie configuré pour extraire de la cavité interne l'énergie dissipée par le dispositif portatif sans perturber la barrière stérile.

14. Instrument chirurgical (6000, 6700) selon la revendication 13, comprenant en outre :
une pluralité d'effecteurs terminaux interchangeables (6008), un premier effecteur terminal interchangeable de la pluralité d'effecteurs terminaux interchangeables comprenant une première interface d'entraînement configurée pour un premier mode de fonctionnement, et un second effecteur terminal interchangeable de la pluralité d'effecteurs terminaux interchangeables comprenant une seconde interface d'entraînement configurée pour un second mode de fonctionnement, le premier mode de fonctionnement étant différent du second mode de fonctionnement ; et
un ensemble d'interface d'entraînement comprenant une interface interne configurée pour venir en prise avec le premier ensemble d'entraînement et une interface externe configurée pour venir en prise avec la première interface d'entraînement et la seconde interface d'entraînement.

15. Instrument chirurgical (6700) selon la revendication 14, l'ensemble adaptateur comprenant en outre une partie proximale et une partie distale couplée de manière rotative à la partie proximale par l'intermédiaire d'une charnière, la partie distale (6704) étant configurée pour se déplacer par rapport à la partie proximale entre une configuration ouverte et une configuration fermée, et le système de gestion de l'énergie comprenant :
un premier dissipateur thermique (6708) couplé à la partie proximale, le premier dissipateur thermique étant configuré pour un contact mécanique avec le dispositif portatif (6702), et
un second dissipateur thermique (6712) couplé à une surface extérieure de la partie distale (6704) du boîtier externe, le second dissipateur thermique étant configuré pour entrer en contact mécanique avec le premier dissipateur thermique sans perturber la barrière stérile lorsque l'ensemble adaptateur est dans la configuration fermée, et le contact mécanique entre le premier dissipateur thermique et le second dissipateur thermique créant un chemin thermiquement conducteur entre le dispositif portatif et le second dissipateur thermique.
